# EUROPÄISCHE PATENTSCHRIFT

(11) **EP 1 651 649 B1**
(45) Veröffentlichungstag und Bekanntmachung des Hinweises auf die Patenterteilung: **01.12.2010**
(21) Anmeldenummer: 04740981.8
(22) Anmeldetag: 14.07.2004
(51) Int. Cl.: C07D 487/04, A61K 31/415

(54) **NEUE CYANOPYRROLIDIDE, VERFAHREN ZU IHRER HERSTELLUNG UND IHRE VERWENDUNG ALS ARZNEIMITTEL**
NOVEL CYANOPYRROLIDIDES, METHODS FOR THE PRODUCTION THEREOF, AND USE OF THE SAME AS MEDICATION
NOUVEAUX CYANOPYRROLIDIDES, PROCEDES POUR LEUR PRODUCTION ET LEUR UTILISATION COMME MEDICAMENT

(30) Priorität: 25.07.2003 DE 10333935; 21.04.2004 DE 102004019276
(43) Veröffentlichungstag der Anmeldung: 03.05.2006
(73) Patentinhaber: Sanofi-Aventis Deutschland GmbH, 65929 Frankfurt am Main (DE)
(72) Erfinder: SCHOENAFINGER, Karl, 63755 Alzenau (DE); WAGNER, Holger, 88400 Biberach/ Mettenberg (DE); JAEHNE, Gerhard, 65929 Frankfurt (DE); GAUL, Holger, 65594 Runkel (DE); BUNING, Christian, 53175 Bonn (DE); TSCHANK, Georg, 55270 Essenheim (DE); WERNER, Ulrich, 56357 Miehlen (DE)
(86) Internationale Anmeldenummer: PCT/EP2004/007761
(87) Internationale Veröffentlichungsnummer: WO 2005/012308

(56) Entgegenhaltungen:
- EP-A- 0 485 219
- WO-A-99/31507

## Beschreibung

Die Erfindung betrifft substituierte Cyanopyrrolidide sowie deren physiologisch verträglichen Salze.

Es sind bereits strukturähnliche Verbindungen im Stand der Technik beschrieben (WO 99/31507) sowie deren Verwendung in einem Screening.

Der Erfindung lag die Aufgabe zugrunde, Verbindungen zur Verfügung zu stellen, die eine therapeutisch verwertbare, Blutzucker senkende Wirkung entfalten und insbesondere zur Behandlung von Diabetes geeignet sind.

Die Erfindung betrifft daher Verbindungen der Formel I,
worin bedeuten 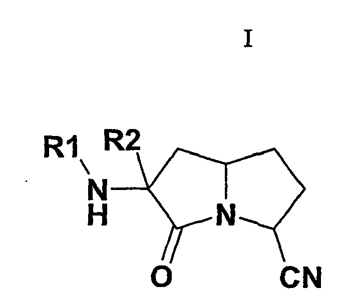
- R1: H, (C₁-C₁₀)-Alkyl, (C₃-C₁₀)-Cycoalkyl, (C₂-C₆)-Alkenyl, (C₂-C₆)-Alkinyl, (C₆- C₁₀)-Aryl, Heterozyklyl, COR3, COOR3, CONR3R4, CN, wobei die Alkyl-, Cycloalkyl-, Alkenyl-, Alkinyl-, Aryl- und Heterozyklyl Reste ein-oder mehrfach substituiert sein können mit F, Cl, Br, I, CN, NO₂, SH, SF₅, OH, (C₁- C₆)-Alkyl, -CF₃, (C₂-C₆)-Alkenyl, (C₂-C₆)-Alkinyl, OR3, OP(O)(OR3)₂, NR3R4, NR3CONR3R4, COR3, OCOR3, OCOOR3, COOR3, CONR3R4, OCONR3R4, (C₁-C₆)-Alkylen-OR3, (C₁-C₆)-Alkylen-NR3R4, (C₁-C₆)-Alkylen-NR3SO₂R4, (C₁-C₆)-Alkylen-SR3, Alkylen-S(O)R3, Alkylen-S(O)₂R3 , Alkylen- S(O)₂NR3R4, (C₁-C₆)-Alkylen-COR3, (C₁-C₆)-Alkylen-COOR3, (C₁-C₆)- Alkylen-CONR3R4, SR3, SOR3, SO₂R3, SO₂NR3R4, NR3SO₂R4, (C₁-C₆)- Alkylen-(C₃-C₁₀)-Cycloalkyl, (C₁-C₆)-Alkylen-(C₆-C₁₀)-Aryl, (C₁-C₆)-Alkylen- Heterozyklyl, (C₃-C₁₀)-Cycloalkyl, (C₆-C₁₀)-Aryl oder Heterozyklyl;
- R2: H, (C₁-C₁₀)-Alkyl, (C₃-C₁₀)-Cycloakyl, (C₂-C₆)-Alkenyl, (C₂-C₆)-Alkinyl, (C₆- C₁₀)-Aryl, Heterozyklyl, COR3, COOR3, CONR3R4, CN, wobei die Alkyl-, Cycloalkyl-, Alkenyl-, Alkinyl-, Aryl- und Heterozyklyl Reste ein-oder mehrfach substituiert sein können mit F, Cl, Br, I, CN, NO₂, SH, SF₅, OH, C₁- C₆)-Alkyl, -CF₃, (C₂-C₆)-Alkenyl, (C₂-C₆)-Alkinyl, OR3, OP(O)(OR3)₂, NR3R4, NR3CONR3R4, COR3, OCOR3, OCOOR3, COOR3, CONR3R4, OCONR3R4, (C₁-C₆)-Alkylen-OR3, (C₁-C₆)-Alkylen-NR3R4, (C₁-C₆)-Alkylen-NR3SO₂R4, (C₁-C₆)-Alkylen-SR3, Alkylen-S(O)R3, Alkylen-S(O)₂R3, Alkylen- S(O)₂NR3R4, (C₁-C₆)-Alkylen-COR3, (C₁-C₆)-Alkylen-COOR3, (C₁-C₆)- Alkylen-CONR3R4, SR3, SOR3, SO₂R3, SO₂NR3R4, NR3SO₂R4, (C₁-C₆)- Alkylen-(C₃-C₁₀)-Cycloalkyl, (C₁-C₆)-Alkylen-(C₆-C₁₀)-Aryl, (C₁-C₆)-Alkylen- Heterozyklyl, (C₃-C₁₀)-Cycloalkyl, (C₆-C₁₀)-Aryl oder Heterozyklyl;
- R3, R4: unabhängig voneinander H, (C₁-C₆)-Alkyl, -CF₃, (C₃-C₁₀)-Cycloalkyl, (C₆-C₁₀)- Aryl, Heterozyklyl, (C₁-C₆)-Alkylen-CONR5R6, CONR5R6, (C₁-C₆)-Alkylen- COOR5, COOR5, COR5, (C₁-C₆)-Alkylen-COR5, (C₁-C₆)-Alkylen-OR5, (C₁- C₆)-Alkylen-NR5R6, (C₁-C₆)-Alkylen-SR5, (C₁-C₆)-Alkylen-S(O)R5, (C₁-C₆)- Alkylen-S(O)₂R5, S(O)R5, S(O)₂R5, (C₁-C₄)-Alkylen-(C₆-C₁₀)-Aryl oder (C₁- C₄)-Alkylen-Heterozyklyl;
- R5, R6: unabhängig voneinander H, (C₁-C₆)-Alkyl, (C₁-C₆)-Alkylen-(C₆-C₁₀)-Aryl, -(C₆- C₁₀)-Aryl, Heterozyklyl, (C₁-C₆)-Akylen-Heterozyklyl;
sowie deren physiologisch verträglichen Salze.

Bevorzugt sind Verbindungen der Formel I, worin ein oder mehrere Reste bedeuten
- R1: H
- R2: H, (C₁-C₁₀)-Alkyl, (C₃-C₁₀)-Cycloalkyl, (C₂-C₆)-Alkenyl, (C₂-C₆)-Alkinyl, (C₆- C₁₀)-Aryl, Heterozyklyl, COR3, COOR3, CONR3R4, CN, wobei die Alkyl-, Cycloalkyl-, Alkenyl-, Alkinyl-, Aryl- und Heterozyklyl Reste ein-oder mehrfach substituiert sein können mit F, Cl, Br, I, CN, NO₂, SH, SF₅, OH, (C₁- C₆)-Alkyl, -CF₃, (C₂-C₆)-Alkenyl, (C₂-C₆)-Alkinyl, OR3, OP(O)(OR3)₂, NR3R4, NR3CONR3R4, COR3, OCOR3, OCOOR3, COOR3, CONR3R4, OCONR3R4, (C₁-C₆)-Alkylen-OR3, (C₁-C₆)-Alkylen-NR3R4, (C₁-C₆)-Alkylen-NR3SO₂R4, (C₁-C₆)-Alkylen-SR3, Alkylen-S(O)R3, Alkylen-S(O)₂R3, Alkylen- S(O)₂NR3R4, (C₁-C₆)-Alkylen-COR3, (C₁-C₆)-Alkylen-COOR3, (C₁-C₆)- Alkylen-CONR3R4, SR3, SOR3, SO₂R3, SO₂NR3R4, NR3SO₂R4, (C₁-C₆)- Alkylen-(C₃-C₁₀)-Cycloalkyl, (C₁-C₆)-Alkylen-(C₆-C₁₀)-Aryl, (C₁-C₆)-Alkylen- Heterozyklyl, (C₃-C₁₀)-Cycloalkyl, (C₆-C₁₀)-Aryl oder Heterozyklyl;
- R3, R4: unabhängig voneinander H, (C₁-C₆)-Alkyl, -CF₃, (C₃-C₁₀)-Cycloalkyl, (C₆-C₁₀)- Aryl, Heterozyklyl, (C₁-C₆)-Alkylen-CONR5R6, CONR5R6, (C₁-C₆)-Alkylen- COOR5, COOR5, COR5, (C₁-C₆)-Alkylen-COR5, (C₁-C₆)-Alkylen-OR5, (C₁- C₆)-Alkylen-NR5R6, (C₁-C₆)-Alkylen-SR5, (C₁-C₆)-Alkylen-S(O)R5, (C₁-C₆)- Alkylen-S(O)₂R5, S(O)R5, S(O)₂R5, (C₁-C₄)-Alkylen-(C₆-C₁₀)-Aryl oder (C₁- C₄)-Alkylen-Heterozyklyl;
- R5, R6: unabhängig voneinander H, (C₁-C₆)-Alkyl, (C₁-C₆)-Alkylen-(C₆-C₁₀)-Aryl, -(C₆- C₁₀)-Aryl, Heterozyklyl, (C₁-C₆)-Alkylen-Heterozyklyl;
sowie deren physiologisch verträglichen Salze.

Weiterhin bevorzugt sind Verbindungen der Formel I, worin ein oder mehrere Reste bedeuten
- R1: H
- R2: (C₁-C₁₀)-Alkyl, (C₃-C₁₀)-Cycloalkyl, (C₂-C₆)-Alkenyl, (C₂-C₆)-Alkinyl, (C₆-C₁₀)- Aryl, Heterozyklyl, COR3, COOR3, CONR3R4, CN, wobei die Alkyl-, Cycloalkyl-, Alkenyl-, Alkinyl-, Aryl- und Heterozyklyl Reste ein-oder mehrfach substituiert sein können mit F, Cl, Br, I, CN, NO₂, SH, SF₅, OH, (C₁- C₆)-Alkyl, -CF₃, (C₂-C₆)-Alkenyl, (C₂-C₆)-Alkinyl, OR3, OP(O)(OR3)₂, NR3R4, NR3CONR3R4, COR3, OCOR3, OCOOR3, COOR3, CONR3R4, OCONR3R4, (C₁-C₆)-Alkylen-OR3, (C₁-C₆)-Alkylen-NR3R4, (C₁-C₆)-Alkylen-NR3SO₂R4, (C₁-C₆)-Alkylen-SR3, Alkylen-S(O)R3, Akylen-S(O)₂R3, Alkylen- S(O)₂NR3R4, (C₁-C₆)-Alkylen-COR3, (C₁-C₆)-Akylen-COOR3, (C₁-C₆)- Alkylen-CONR3R4, SR3, SOR3, SO₂R3, SO₂NR3R4, NR3SO₂R4, (C₁-C₆)- Alkylen-(C₃-C₁₀)-Cycloalkyl, (C₁-C₆)-Alkylen-(C₆-C₁₀)-Aryl, (C₁-C₆)-Alkylen- Heterozyklyl, (C₃-C₁₀)-Cycloalkyl, (C₆-C₁₀)-Aryl oder Heterozyklyl;
- R3, R4: unabhängig voneinander H, (C₁-C₆)-Alkyl, -CF₃, (C₃-C₁₀)-Cycloalkyl, (C₆-C₁₀)- Aryl, Heterozyklyl, (C₁-C₆)-Alkylen-CONR5R6, CONR5R6, (C₁-C₆)-Alkylen- COOR5, COOR5, COR5, (C₁-C₆)-Alkylen-COR5, (C₁-C₆)-Alkylen-OR5, (C₁- C₆)-Alkylen-NR5R6, (C₁-C₆)-Alkylen-SR5, (C₁-C₆)-Alkylen-S(O)R5, (C₁-C₆)- Alkylen-S(O)₂R5, S(O)R5, S(O)₂R5, (C₁-C₄)-Alkylen-(C₆-C₁₀)-Aryl oder (C₁- C₄)-Alkylen-Heterozyklyl;
- R5, R6: unabhängig voneinander H, (C₁-C₆)-Alkyl,(C₁-C₆)-Alkylen-(C₆-C₁₀)-Aryl, -(C₆- C₁₀)-Aryl, Heterozyklyl, (C₁-C₆)-Alkylen-Heterozyklyl;
sowie deren physiologisch verträglichen Salze.

Weiterhin bevorzugt sind Verbindungen der Formel I, worin ein oder mehrere Reste bedeuten
- R1: H;
- R2: (C₁-C₁₀)-Alkyl, (C₃-C₁₀)-Cycloalkyl, (C₂-C₆)-Alkenyl, (C₂-C₆)-Alkinyl, (C₆-C₁₀)- Aryl, ein Pyrrolidino-, Piperidino-, Hexamethylenimino-, Morpholin-, Piperazino-, Thiomorpholino- oder Homopiperäzino-Rest; wobei die Alkyl-, Cycloalkyl-, Alkenyl-, Alkinyl-, Pyrrolidino-, Piperidino-, Hexamethylenimino-, Morpholino-, Piperazino-, Thiomorpholino-, Homopiperazino- und Aryl-Reste ein-oder mehrfach substituiert sein können mit F, Cl, Br, CN, SF₅, OH, (C₁-C₆)- Alkyl, -CF₃, (C₂-C₆)-Alkenyl, OR3, NR3R4, NR3CONR3R4, COR3, OCOR3, CO₂R3, CONR3R4, OCONR3R4, (C₁-C₆)-Alkylen-OR3, (C₁-C₆)-Alkylen- NR3R4, (C₁-C₆)-Alkylen-NR3SO₂R4, (C₁-C₆)-Alkylen-SR3, Alkylen-S(O)R3, Alkylen-S(O)₂R3, Alkylen-S(O)₂NR3R4, (C₁-C₆)-Alkylen-COR3, (C₁-C₆)- Alkylen-CO₂R3, (C₁-C₆)-Alkylen-CONR3R4, SR3, SOR3, SO₂R3, SO₂NR3R4, NR3SO₂R4, (C₁-C₆)-Alkylen-(C₃-C₁₀)-Cycloalkyl, (C₁-C₆)-Alkylen-(C₆-C₁₀)- Aryl, (C₁-C₆)-Alkylen-Heterozyklyl, (C₃-C₁₀)-Cycoalkyl, (C₆-C₁₀)-Aryl, das mit F, Cl, Br, I, CN, OH, -CF₃, (C₁-C₆)-Alkyl, OR3, NR3R4, COR3, CO2R3, CONR3R4 ein oder mehrfach substituiert sein kann, oder Heterozyklyl, das mit F, Cl, Br, CN, NO₂, OH, -CF₃, (C₁-C₆)-Alkyl, OR3, NR3R4, COR3, CO2R3, CONR3R4 ein oder mehrfach substituiert sein kann;
- R3, R4: unabhängig voneinander H, (C₁-C₆)-Alkyl, -CF₃, (C₃-C₁₀)-Cycloalkyl, (C₆-C₁₀)- Aryl, Heterozyklyl, (C₁-C₆)-Alkylen-CONR5R6, (C₁-C₆)-Alkylen-COOR5, (C₁- C₆)-Alkylen-COR5, (C₁-C₆)-Alkylen-OR5, (C₁-C₆)-Alkylen-NR5R6, (C₁-C₆)- Alkylen-SR5, (C₁-C₆)-Alkylen-S(O)R5, (C₁-C₆)-Alkylen-S(O)₂R5, (C₁-C₄)- Alkylen-(C₆-C₁₀)-Aryl oder (C₁-C₄)-Alkylen-Heterozyklyl;
- R5, R6: unabhängig voneinander H, (C₁-C₆)-Alkyl, (C₃-C₁₀)-Cycloalkyl, (C₁-C₆)- Alkylen-(C₆-C₁₀)-Aryl, -(C₆-C₁₀)-Aryl, Heterozyklyl, (C₁-C₆)-Alkylen-(C₃-C₁₀)- Heterozyklyl;
sowie deren physiologisch verträglichen Salze.

Weiterhin bevorzugt sind Verbindungen der Formel I, worin ein oder mehrere Reste bedeuten
- R1: H;
- R2: (C₁-C₁₀)-Alkyl, (C₃-C₁₀)-Cycloalkyl, Phenyl, (C₁-C₆)-Alkylen-Phenyl, ein Pyrrolidino-, Piperidino-, Hexamethylenimino-, Morpholino-, Piperazino-, Thiomorpholino- oder Homopiperazino-Rest;
sowie deren physiologisch verträglichen Salze.

Die Erfindung betrifft Verbindungen der Formel I in Form all ihrer stereoisomeren Formen wie Racemate, racemischen und enantiomeren Mischungen und reinen Enantiomeren und Diastereomeren.

Besonders bevorzugt sind Verbindungen der Formel I, 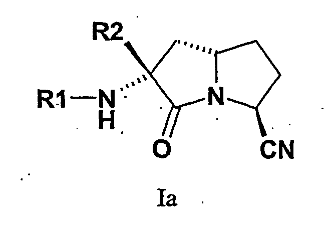 die die angegebene diastereomere Form Ia aufweisen.

Die Erfindung betrifft Verbindungen der Formel I in Form all ihrer stereoisomeren Formen wie Racemate, racemischen und enantiomeren Mischungen und reinen Enantiomeren und Diastereomeren.

Können Reste oder Substituenten mehrfach in den Verbindungen der Formel I auftreten, so können sie alle unabhängig voneinander die angegebenen Bedeutungen haben und gleich oder verschieden sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Salze von Trometamol (2-Amino-2-hydroximethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze, Solvate und physiologisch funktionellen Derivate wie hierin beschrieben.

Unter einem Alkylrest wird eine geradkettige oder verzweigte Kohlenwasserstoffkette mit einem oder mehreren Kohlenstoffen verstanden, wie z.B. Methyl, Ethyl, Propyl, Butyl, Hexyl, Isopropyl, Isobutyl, Neopentyl, tert.-Butyl, Hexyl.
Die Alkylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₂-C₆)-Alkinyl, Aryl, Heterozyklyl, O-(C₁-C₆)-Alkyl, O-COO-(C₁-C₆)-Alkyl, O-CO-(C₁-C₆)-Alkyl, O-CO-(C₁-C₆)-Aryl, O-CO-(C₁-C₆)-Heterozyklyl, PO₃H₂, P(O)(OAlkyl)₂, (C₁-C₆)-Alkylen-P(O)(OAlkyl)₂, O-P(O)(OH)₂, O-P(O)(OAlkyl)₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterozyklyl, SO-(C₁-C₆)-Alkyl, SO-(CH₂)ₙ-Aryl, SO-(CH₂)ₙ-Heterozyklyl, SO₂-(C₁-C₆)-Alkyl, SO₂-(CH₂)ₙ-Aryl, SO₂-(CH₂)ₙ-Heterozyklyl, SO₂-NH(CH₂)ₙ-Aryl, SO₂-NH(CH₂)ₙ-Heterozyklyl, SO₂-N[((C₁-Cs)-Alkyl)(CH₂)ₙ-Aryl], SO₂-N[((C₁-C₆)-Alkyl)(CH₂)ₙ-Heterozyklyl], SO₂-N((CH₂)ₙ-Acyl)₂, , SO₂-N((CH₂)ₙ-(Heterozyklyl))₂, wobei n = 0 - 6 sein kann und der Aryl- oder Heterozyklyl- Rest bis zu dreifach mit F, Cl, Br, OH, CF₃, SF₅, NO₂, CN, OCF₃, O-(C₁-C₆)-Alkyl, (C₁-C₆)-Alkyl, NH₂ substituiert sein kann;
C(NH)(NH₂), NH₂, NH-(C₁-C₆)-Alkyl, N((C₁-C₆)-Akyl)₂, NH(C₁-C₇)-Acyl, NH-CO-(C₁-C₆)-Alkyl, NH-COO-(C₁-C₆)-Alkyl, NH-CO-Aryl, NH-CO-Heterozyklyl, NH-COO-Aryl, NH-COO-Heterozyklyl, NH-CO-NH-(C₁-C₆)-Alkyl), NH-CO-NH-Aryl, NH-CO-NH-Heterozyklyl, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterozyklyl, N[(C₁-C₆)-Alkyl]-COO-Aryl, N[(C₁-C₆)-Alkyl]-COO-Heterozyklyl, N[(C₁-C₆)-Alkyl]-CO-NH-(C₁-C₆)-Akyl), N[(C₁-C₆)-Alkyl]-CO-NH-Aryl, N[(C₁-C₆)-Alkyl]-CO-NH-Heterozyklyl, N[(C₁-C₆)-Alkyl]-CO-N((C₁-C₆)-Alkyl)₂, N[(C₁-C₆)-Alkyl]-CO-N((C₁-C₆)-Alkyl)-Aryl, N[(C₁-C₆)-Alkyl]-CO-N((C₁-C₆)-Alkyl)-Heterozyklyl, N[(C₁-C₆)-Alkyl]-CO-N(Aryl)₂, N[(C₁-C₆)-Alkyl]-CO-N(Heterozyklyl)₂, N(Aryl)-CO-(C₁-C₆)-Alkyl, N(Heterozyklyl)-CO-(C₁-C₆)-Alkyl, N(Aryl)-COO-(C₁-C₆)-Alkyl, N(Heterozyklyl)-COO-(C₁-C₆)-Alkyl, N(Aryl)-CO-Aryl, N(Heterozyklyl)-CO-Aryl, N(Aryl)-COO-Aryl, N(Heterozyklyl)-COO-Aryl, N(Aryl)-CO-NH-(C₁-C₆)-Alkyl, N(Heterozyklyl)-CO-NH-(C₁-C₆)-Alkyl, N(Aryl)-CO-NH-Aryl, N(Heterozyklyl)-CO-NH-Aryl, N(Aryl)-CO-N((C₁-C₆)-Alkyl)₂, N(Heterozyklyl)-CO-N((C₁-C₆)-Alkyl)₂, N(Aryl)-CO-N[(C₁-C₆)-Alkyl]-Aryl, N(Heterozyklyl)-CO-N[(C₁-C₆)-Alkyl]-Aryl, N(Aryl)-CO-N(Aryl)₂, N(Heterozyklyl)-CO-N(Aryl)₂, Aryl, O-(CH₂)ₙ-Aryl, O-(CH₂)ₙ-Heterozyklyl, wobei n = 0 - 6 sein kann, wobei der Aryl- oder Heterozyklyl- Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH₂.

Unter einem Alkenylrest wird eine geradkettige oder verzweigte Kohlenwasserstoffkette mit zwei oder mehreren Kohlenstoffen sowie einer oder mehreren Doppelbindungen verstanden, wie z.B. Vinyl, Allyl, Pentenyl.
Die Alkenylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₁-C₁₀)-Alkyl, (C₂-C₆)-Alkinyl, Aryl, Heterozyklyl, O-(C₁-C₆)-Alkyl O-CO-(C₁-C₆)-Alkyl, O-CO-(C₁-C₆)-Aryl, O-CO-(C₁-C₆)-Heterozyklyl,;
PO₃H₂, P(O)(OAlkyl)₂, (C₁-C₆)-Alkylen-P(O)(OAlkyl)₂, O-P(O)(OH)₂, O-P(O)(OAlkyl)₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterozyklyl, SO-(C₁-C₆)-Alkyl, SO-(CH₂)ₙ-Aryl, SO-(CH₂)ₙ-Heterozyklyl, SO₂-(C₁-C₆)-Alkyl, SO₂-(CH₂)ₙ-Aryl,SO₂-(CH₂)ₙ-Heterozyklyl, SO₂-NH(CH₂)ₙ-Aryl, SO₂-NH(CH₂)ₙ-Heterozyklyl, SO₂-N((C₁-C₆)-Alkyl)(CH₂)ₙ-Aryl, SO₂-N((C₁-C₆)-Alkyl)(CH₂)ₙ-Heterozyklyl, SO₂-N((CH₂)ₙ-Aryl)₂, , SO₂-N((CH₂)ₙ-(Heterozyklyl)₂ wobei n = 0 - 6 sein kann und der Arylrest oder heterozyklische Rest bis zu zweifach mit F, Cl, Br, OH, CF₃, SF₅, NO₂, CN, OCF₃, O-(C₁-C₆)-Alkyl, (C₁-C₆)-Alkyl, NH₂ substituiert sein kann;
C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterozyklyl, NH-COO-Aryl, NH-COO-Heterozyklyl, NH-CO-NH-(C₁-C₆)-Alkyl), NH-CO-NH-Aryl, NH-CO-NH-Heterozyklyl, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterozyklyl, N[(C₁-C₆)-Alkyl]-COO-Aryl, N[(C₁-C₆)-Alkyl]-COO-Heterozyklyl, N[(C₁-C₆)-Alkyl]-CO-NH-(C₁-C₆)-Alkyl), N[(C₁-C₆)-Alkyl]-CO-NH-Aryl, N[(C₁-C₆)-Alkyl]-CO-NH-Heterozyklyl, N[(C₁-C₆)-Alkyl]-CO-N((C₁-C₆)-Alkyl)₂, N[(C₁-C₆)-Alkyl]-CO-N((C₁-C₆)-Alkyl)-Aryl, N[(C₁-C₆)-Alkyl]-CO-N((C₁-C₆)-Alkyl)-Heterozyklyl, N[(C₁-C₆)-Alkyl]-CO-N(Aryl)₂, N[(C₁-C₆)-Alkyl]-CO-N(Heterozyklyl)₂, N(Aryl)-CO-(C₁-C₆)-Alkyl, N(Heterozyklyl)-CO-(C₁-C₆)-Alkyl, N(Aryl)-COO-(C₁-C₆)-Alkyl, N(Heterozyklyl)-COO-(C₁-C₆)-Alkyl, N(Aryl)-CO-Aryl, N(Heterozyklyl)-CO-Aryl, N(Aryl)-COO-Aryl, N(Heterozyklyl)-COO-Aryl, N(Aryl)-CO-NH-(C₁-C₆)-Alkyl, N(Heterozyklyl)-CO-NH-(C₁-C₆)-Alkyl, N(Aryl)-CO-NH-Aryl, N(Heterozyklyl)-CO-NH-Aryl, N(Aryl)-CO-N((C₁-C₆)-Alkyl)₂, N(Heterozyklyl)-CO-N((C₁-C₆)-Alkyl)₂, N(Aryl)-CO-N[(C₁-C₆)-Alkyl]-Aryl, N(Heterozyklyl)-CO-N[(C₁-C₆)-Alkyl]-Aryl, N(Aryl)-CO-N(Aryl)₂, N(Heterozyklyl)-CO-N(Aryl)₂, Aryl, O-(CH₂)ₙ-Aryl, O-(CH₂)ₙ-Heterozyklyl, wobei n = 0 - 6 sein kann, wobei der Aryl- oder Heterozyklyl- Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₄)-Alkyl, N((C₁-C₆)-Alkyl)₂, SF₅, SO₂-CH₃, COOH, COO-(C₁-C₆)-Alkyl, CONH₂.

Unter einem Alkinylrest wird eine geradkettige oder verzweigte Kohlenwasserstoffkette mit zwei oder mehreren Kohlenstoffen sowie einer oder mehreren Dreifachbindungen verstanden, wie z.B. Ethinyl, Propinyl, Hexinyl.
Die Alkinylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Akyl, CON[(C₁-C₆)Alkyl]₂, Cycloalkyl, (C₂-C₆)-Alkenyl, (C₁-C₁₀)-Alkyl, O-(C₁-C₆)-Alkyl O-CO-(C₁-C₆)-Alkyl, O-CO-(C₁-C₆)-Aryl, O-CO-(C₁-C₆)-Heterozyklyl;
PO₃H₂, P(O)(OAlkyl)₂, (C₁-C₆)-Alkylen-P(O)(OAlkyl)₂, O-P(O)(OH)₂, O-P(O)(OAlkyl)₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterozyklyl, SO-(C₁-C₆)-Alkyl, SO-(CH₂)ₙ-Aryl, SO-(CH₂)ₙ-Heterozyklyl, SO₂-(C₁-C₆)-Alkyl, SO₂-(CH₂)ₙ-Aryl, SO₂-(CH₂)ₙ-Heterozyklyl, SO₂-NH(CH₂)ₙ-Aryl, SO₂-NH(CH₂)ₙ-Heterozyklyl, SO₂-N((C₁-C₆)-Alkyl)(CH₂)ₙ-Aryl, SO₂-N((C₁-C₆)-Alkyl)(CH₂)n-Heterozyklyl, SO₂-N((CH₂)ₙ-Aryl)₂, , SO2-N((CH₂)ₙ-(Heterozyklyl)₂ wobei n = 0 - 6 sein kann und der Arylrest oder Heterozyklische Rest bis zu zweifach mit F, Cl, Br, OH, CF₃, SF₅, NO₂, CN, OCF₃, O-(C₁-C₆)-Alkyl, (C₁-C₆)-Alkyl, NH₂ substituiert sein kann;
C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterozyklyl, NH-COO-Aryl, NH-COO-Heterozyklyl, NH-CO-NH-(C₁-C₆)-Alkyl), NH-CO-NH-Aryl, NH-CO-NH-Heterozyklyl, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterozyklyl, N[(C₁-C₆)-Alkyl]-COO-Aryl, N[(C₁-C₆)-Alkyl]-COO-Heterozyklyl, N[(C₁-C₆)-Alkyl]-CO-NH-(C₁-C₆)-Alkyl), N[(C₁-C₆)-Alkyl]-CO-NH-Aryl, N[(C₁-C₆)-Alkyl]-CO-NH-Heterozyklyl, N[(C₁-C₆)-Alkyl]-CO-N((C₁-C₆)-Alkyl)₂, N[(C₁-C₆)-Alkyl)-CO-N((C₁-C₆)-Alkyl)-Aryl, N[(C₁-C₆)-Alkyl]-CO-N((C₁-C₆)-Alkyl)-Heterozyklyl, N[(C₁-C₆)-Alkyl]-CO-N(Aryl)₂, N[(C₁-C₆)-Alkyl]-CO-N(Heterozyklyl)₂, N(Aryl)-CO-(C₁-C₆)-Alkyl, N(Heterozyklyl)-CO-(C₁-C₆)-Alkyl, N(Aryl)-COO-(C₁-C₆)-Alkyl, N(Heterozyklyl)-COO-(C₁-C₆)-Alkyl, N(Aryl)-CO-Aryl, N(Heterozyklyl)-CO-Aryl, N(Aryl)-COO-Aryl, N(Heterozyklyl)-COO-Aryl, N(Aryl)-CO-NH-(C₁-C₆)-Alkyl, N(Heterozyklyl)-CO-NH-(C₁-C₆)-Alkyl, N(Aryl)-CO-NH-Aryl, N(Heterozyklyl)-CO-NH-Aryl, N(Aryl)-CO-N((C₁-C₆)-Alkyl)₂, N(Heterozyklyl)-CO-N((C₁-C₆)-Alkyl)₂, N(Aryl)-CO-N[(C₁-C₆)-Alkyl]-Aryl, N(Heterozyklyl)-CO-N[(C₁-C₆)-Alkyl]-Aryl, N(Aryl)-CO-N(Aryl)₂, N(Heterozyklyl)-CO-N(Aryl)₂, Aryl, O-(CH₂)ₙ-Aryl, O-(CH₂)ₙ-Heterozyklyl, wobei n = 0 - 6 sein kann, wobei der Aryl- oder Heterozyklyl- Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH₂.
Unter einem Arylrest wird ein Phenyl, Naphthyl-, Biphenyl-, Tetrahydronaphthyl-, alpha- oder beta-Tetralon-, Indanyl- oder Indan-1-on-ylrest verstanden.
Die Arylreste können ein oder mehrfach mit geeigneten Gruppen substituiert sein, wie z.B.: F, Cl, Br, I, CF₃, NO₂, SF₅, N₃, CN, COOH, COO(C₁-C₆)Alkyl, CONH₂, CONH(C₁-C₆)Alkyl, CON[(C₁-C₆)Alkyl]₂, Cycloalkyl, (C₁-C₁₀)-Alkyl, (C₂-C₆)-Alkenyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterozyklyl,;
PO₃H₂, P(O)(OAlkyl)₂, (C₁-C₆)-Alkylen-P(O)(OAlkyl)₂, O-P(O)(OH)₂, O-P(O)(OAlkyl)₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Aryl, S-(CH₂)ₙ-Heterozyklyl, SO-(C₁-C₆)-Alkyl, SO-(CH₂)ₙ-Aryl, SO-(CH₂)ₙ-Heterozyklyl, SO₂-(C₁-C₆)-Alkyl, SO₂-(CH₂)ₙ-Aryl, SO₂-(CH₂)ₙ-Heterozyklyl, SO₂-NH(CH₂)ₙ-Aryl, SO₂-NH(CH₂)ₙ-Heterozyklyl, SO₂-N((C₁-C₆)-Alkyl)(CH₂)ₙ-Aryl, SO₂-N((C₁-C₆)-Alkyl)(CH₂)ₙ-Heterozyklyl, SO₂-N((CH₂)ₙ-Aryl)₂, , SO₂-N((CH₂)ₙ-(Heterozyklyl)₂ wobei n = 0 - 6 sein kann und der Arylrest oder Heterozyklische Rest bis zu zweifach mit F, Cl, Br, OH, CF₃, NO₂, SF₅, CN, OCF₃, O-(C₁-C₆)-Alkyl, (C₁-C₆)-Alkyl, NH₂ substituiert sein kann;
C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterozyklyl, NH-COO-Aryl, NH-COO-Heterozyklyl, NH-CO-NH-(C₁-C₆)-Alkyl), NH-CO-NH-Aryl, NH-CO-NH-Heterozyklyl, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterozyklyl, N[(C₁-C₆)-Alkyl]-COO-Aryl, N[(C₁-C₆)-Alkyl]-COO-Heterozyklyl, N[(C₁-C₆)-Alkyl]-CO-NH-(C₁-C₆)-Alkyl), N[(C₁-C₆)-Alkyl]-CO-NH-Aryl, N[(C₁-C₆)-Alkyl]-CO-NH-Heterozyklyl, N[(C₁-C₆)-Alkyl]-CO-N((C₁-C₆)-Alkyl)₂, N[(C₁-C₆)-Alkyl]-CO-N((C₁-C₆)-Alkyl)-Aryl, N[(C₁-C₆)-Alkyl]-CO-N((C₁-C₆)-Alkyl)-Heterozyklyl, N[(C₁-C₆)-Alkyl]-CO-N(Aryl)₂, N[(C₁-C₆)-Alkyl]-CO-N(Heterozyklyl)₂, N(Aryl)-CO-(C₁-C₆)-Alkyl, N(Heterozyklyl)-CO-(C₁-C₆)-Alkyl, N(Aryl)-COO-(C₁-C₆)-Alkyl, N(Heterozyklyl)-COO-(C₁-C₆)-Alkyl, N(Aryl)-CO-Aryl, N(Heterozyklyl)-CO-Aryl, N(Aryl)-COO-Aryl, N(Heterozyklyl)-COO-Aryl, N(Aryl)-CO-NH-(C₁-C₆)-Alkyl, N(Heterozyklyl)-CO-NH-(C₁-C₆)-Alkyl, N(Aryl)-CO-NH-Aryl, N(Heterozyklyl)-CO-NH-Aryl, N(Aryl)-CO-N((C₁-C₆)-Alkyl)₂, N(Heterozyklyl)-CO-N((C₁-C₆)-Alkyl)₂, N(Aryl)-CO-N[(C₁-C₆)-Alkyl]-Aryl, N(Heterozyklyl)-CO-N[(C₁-C₆)-Alkyl]-Aryl, N(Aryl)-CO-N(Aryl)₂, N(Heterozyklyl)-CO-N(Aryl)₂, Aryl, O-(CH₂)ₙ-Aryl, O-(CH₂)ₙ-Heterozyklyl, wobei n = 0 - 6 sein kann, wobei der Aryl- oder Heterozyklyl- Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH₂.

Unter einem Cycloalkylrest wird ein einen oder mehrere Ringe enthaltendes Ringssystem, welches gesättigt oder partiell ungesättigt (mit einer oder zwei Doppelbindungen) vorliegt, verstanden, das ausschließlich aus Kohlenstoffatomen aufgebaut ist, wie z.B. Cyclopropyl, Cyclopentyl, Cyclopentenyl, Cyclohexyl oder Adamantyl.
Die Cycloalkylrestereste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₁-C₁₀)-Alkyl, (C₂-C₆)-Alkenyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterozyklyl,; PO₃H₂, P(O)(OAlkyl)₂, (C₁-C₆)-Alkylen-P(O)(OAlkyl)₂, O-P(O)(OH)₂, O-P(O)(OAlkyl)₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterozyklyl, SO-(C₁-C₆)-Alkyl, SO-(CH₂)ₙ-Aryl, SO-(CH₂)ₙ-Heterozyklyl, SO₂-(C₁-C₆)-Alkyl, SO₂-(CH₂)ₙ-Aryl, SO₂-(CH₂)ₙ-Heterozyklyl, SO₂-NH(CH₂)ₙ-Aryl, SO₂-NH(CH₂)ₙ-Heterozyklyl, SO₂-N((C₁-C₆)-Alkyl)(CH₂)ₙ-Aryl, SO₂-N((C₁-C₆)-Alkyl)(CH₂)ₙ-Heterozyklyl, SO₂-N((CH₂)ₙ-Aryl)₂, , SO₂-N((CH₂)ₙ-(Heterozyklyl)₂ wobei n = 0 - 6 sein kann und der Arylrest oder Heterozyklische Rest bis zu zweifach mit F, Cl, Br, OH, CF₃, SF₅, NO₂, CN, OCF₃, O-(C₁-C₆)-Alkyl, (C₁-C₆)-Alkyl, NH₂ substituiert sein kann;
C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterozyklyl, NH-COO-Aryl, NH-COO-Heterozyklyl, NH-CO-NH-(C₁-C₆)-Alkyl), NH-CO-NH-Aryl, NH-CO-NH-Heterozyklyl, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterozyklyl, N[(C₁-C₆)-Alkyl]-COO-Aryl, N[(C₁-C₆)-Alkyl]-COO-Heterozyklyl, N[(C₁-C₆)-Alkyl]-CO-NH-(C₁-C₆)-Alkyl), N[(C₁-C₆)-Alkyl]-CO-NH-Aryl, N[(C₁-C₆)-Alkyl]-CO-NH-Heterozyklyl, N[(C₁-C₆)-Alkyl]-CO-N((C₁-C₆)-Alkyl)₂, N[(C₁-C₆)-Alkyl]-CO-N((C₁-C₆)-Alkyl)-Aryl, N[(C₁-C₆)-Alkyl]-CO-N((C₁-C₆)-Alkyl)-Heterozyklyl, N[(C₁-C₆)-Alkyl]-CO-N(Aryl)₂, N[(C₁-C₆)-Alkyl]-CO-N(Heterozyklyl)₂, N(Aryl)-CO-(C₁-C₆)-Alkyl, N(Heterozyklyl)-CO-(C₁-C₆)-Alkyl, N(Aryl)-COO-(C₁-C₆)-Alkyl, N(Heterozyklyl)-COO-(C₁-C₆)-Alkyl, N(Aryl)-CO-Aryl, N(Heterozyklyl)-CO-Aryl, N(Aryl)-COO-Aryl, N(Heterozyklyl)-COO-Aryl, N(Aryl)-CO-NH-(C₁-C₆)-Alkyl, N(Heterozyklyl)-CO-NH-(C₁-C₆)-Alkyl, N(Aryl)-CO-NH-Aryl, N(Heterozyklyl)-CO-NH-Aryl, N(Aryl)-CO-N((C₁-C₆)-Alkyl)₂, N(Heterozyklyl)-CO-N((C₁-C₆)-Alkyl)₂, N(Aryl)-CO-N[(C₁-C₆)-Alkyl]-Aryl, N(Heterozyklyl)-CO-N[(C₁-C₆)-Alkyl]-Aryl, N(Aryl)-CO-N(Aryl)₂, N(Heterozyklyl)-CO-N(Aryl)₂, Aryl, O-(CH₂)ₙ-Aryl, O-(CH₂)ₙ-Heterozyklyl, wobei n = 0 - 6 sein kann, wobei der Aryl- oder Heterozyklyl- Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH₂.

Unter Heterozyklyl bzw. heterozyklischer Rest werden Ringe und Ringsysteme verstanden, die außer Kohlenstoff noch Heteroatome, wie zum Beispiel Stickstoff, Sauerstoff oder Schwefel enthalten. Ferner gehören auch Ringsysteme zu dieser Definition, worin der Heterocylus bzw. der heterozyklische Rest mit Benzolkernen kondensiert ist.

Geeignete Heterozyklyl- bzw. "heterozyklische Reste" sind Acridinyl, Benzimidazolyl, Benzofuryl, Benzothienyl, Benzothiophenyl, Benzoxazolyl, Benzthiazolyl, Benztriazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, 2H,6H-1,5,2-Dithiazinyl, Dihydrofuro[2,3-b]-Tetrahydrofuran, Furyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolyl, Isoxazolyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl; Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyrazolidinyl, Pyrazolinyl, Pyrazolyl, Pyridazinyl, Pyridooxazole, Pyridoimidazole, Pyridothiazol, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, 6H-1,2,5-Thiadazinyl, Thiazolyl, 1,2,3-Thiadiazolyl, 1,2,4-Thiadiazolyl, 1,2,5-Thiadiazolyl, 1,3,4-Thiadiazolyl, Thienyl, Triazolyl, Tetrazolyl, Aziridinyl, Azetininyl, Azepanyl, Azocanyl und Xanthenyl.

Pyridyl steht sowohl für 2-, 3- als auch 4-Pyridyl. Thienyl steht sowohl für 2- als auch 3-Thienyl. Furyl steht sowohl für 2- als auch 3-Furyl.

Umfasst sind weiterhin die entsprechenden N-Oxide dieser Verbindungen, also z.B. 1-Oxy-2-, 3- oder 4-Pyridyl.

Umfasst sind weiterhin ein oder mehrfach benzoannelierte Derivate dieser Heterozyklen.

Die heterozyklischen Ringe bzw. Heterozyklische Reste können ein oder mehrfach mit geeigneten Gruppen substituiert sein, wie z.B: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₁-C₁₀)-Alkyl, (C₂-C₆)-Alkenyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterozyklyl;
PO₃H₂, P(O)(OAlkyl)₂, (C₁-C₆)-Alkylen-P(O)(OAlkyl)₂, O-P(O)(OH)₂, O-P(O)(OAlkyl)₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterozyklyl, SO-(C₁-C₆)-Alkyl, SO-(CH₂)ₙ-Aryl, SO-(CH₂)ₙ-Heterozyklyl, SO₂-(C₁-C₆)-Alkyl, SO₂-(CH₂)ₙ-Aryl, SO₂-(CH₂)ₙ-Heterozyklyl, SO₂-NH(CH₂)ₙ-Aryl, SO₂-NH(CH₂)ₙ-Heterozyklyl, SO₂-N(C₁-C₆)-Alkyl)(CH₂)ₙ-Aryl, SO₂-N(C₁-C₆)-Alkyl)(CH₂)ₙ-Heterozyklyl, SO₂-N((CH₂)ₙ-Aryl)₂,, SO₂-N((CH₂)ₙ-(Heterozyklyl)₂ wobei n = 0 - 6 sein kann und der Arylrest oder heterozyklische Rest bis zu zweifach mit F, Cl, Br, OH, CF₃, SF₅, NO₂, CN, OCF₃, O-(C₁-C₆)-Alkyl, (C₁-C₆)-Alkyl, NH₂ substituiert sein kann;
C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterozyklyl, NH-COO-Aryl, NH-COO-Heterozyklyl, NH-CO-NH-(C₁-C₆)-Alkyl), NH-CO-NH-Aryl, NH-CO-NH-Heterozyklyl, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterozyklyl, N[(C₁-C₆)-Alkyl]-COO-Aryl, N[(C₁-C₆)-Alkyl]-COO-Heterozyklyl, N[(C₁-C₆)-Alkyl]-CO-NH-(C₁-C₆)-Alkyl), N[(C₁-C₆)-Alkyl]-CO-NH-Aryl, N[(C₁-C₆)-Alkyl]-CO-NH-Heterozyklyl, N[(C₁-C₆)-Alkyl]-CO-N((C₁-C₆)-Alkyl)₂, N[(C₁-C₆)-Alkyl]-CO-N((C₁-C₆)-Alkyl)-Aryl, N[(C₁-C₆)-Alkyl]-CO-N((C₁-C₆)-Alkyl)-Heterozyklyl, N[(C₁-C₆)-Alkyl]-CO-N(Aryl)₂, N[(C₁-C₆)-Alkyl]-CO-N(Heterozyklyl)₂, N(Aryl)-CO-(C₁-C₆)-Alkyl, N(Heterozyklyl)-CO-(C₁-C₆)-Alkyl, N(Aryl)-COO-(C₁-C₆)-Alkyl, N(Heterozyklyl)-COO-(C₁-C₆)-Alkyl, N(Aryl)-CO-Aryl, N(Heterozyklyl)-CO-Aryl, N(Aryl)-COO-Aryl, N(Heterozyklyl)-COO-Aryl, N(Aryl)-CO-NH-(C₁-C₆)-Alkyl, N(Heterozyklyl)-CO-NH-(C₁-C₆)-Alkyl, N(Aryl)-CO-NH-Aryl, N(Heterozyklyl)-CO-NH-Aryl, N(Aryl)-CO-N((C₁-C₆)-Alkyl)₂, N(Heterozyklyl)-CO-N((C₁-C₆)-Alkyl)₂, N(Aryl)-CO-N[(C₁-C₆)-Alkyl]-Aryl, N(Heterozyklyl)-CO-N[(C₁-C₆)-Alkyl]-Aryl, N(Aryl)-CO-N(Aryl)₂, N(Heterozyklyl)-CO-N(Aryl)₂, Aryl, O-(CH₂)ₙ-Aryl, O-(CH₂)ₙ-Heterozyklyl, wobei n = 0 - 6 sein kann, wobei der Aryl- oder Heterozyklyl- Rest ein bis 3-fach substituiert sein kann mit F, Cl, Br, I, OH, CF₃, NO₂, CN, OCF₃, SF5, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH₂.

Die Verbindungen der Formel 1 lassen sich nach an sich bekannten Methoden herstellen. So kann man ausgehend von kommerziell erhältlicher S-Pyroglutaminsäure **2** durch Schützung (z.B. mit tert.-Butylacetat und Säure) den tert.-Butylester **3'** erhalten. Durch Boc-Schützung erhält man daraus die Verbindung **4** (siehe auch. Chem. Soc., Perkin Trans 1 1996, 507-514). Umsetzung mit geeigneten Reduktionsmitteln (z. B. Lithium-triethyl-borhydrid) ergibt das Aminal **5.** Durch Olefinierung mit Phosphonoessigsäuretriethylester unter basischen Bedingungen (z. B. NaH) erhält man das Pyrrolidin **6** (Tetrahedron 2000, 4289-4298). Dieser Baustein kann dann durch Reduktion (z. B. mit Lithiumborhydrid) und Oxidation (z. B. Swem-Oxidation J. Org. Chem. 1976, 41, 957) in den Aldehyd **7** (R = H) überführt werden. Alternativ dazu werden durch Umsetzung zum Weinreb Amid (z. B. mit N,O-Dimethyl-hydroxylamin Hydrochlorid in Anwesenheit von Trimethylaluminium) und nachfolgende Addition von geeigneten metallorganischen Reagenzien (Met = Li, MgX wobei X = Cl, Br, I) die Ketone **7** (R ≠ H) erhalten. Anschließende Strecker Synthese (z. B. mit Benzylamin und Trimethylsilylcyanid) ergibt dann die Nitrile **8.** Die Nitrilgruppe wird zum Amid umgesetzt (z. B. mit Kaliumcarbonat in einer Mischung aus Dimethylsulfoxid, Wasser und Wasserstoffperoxid), wobei die Verbindungen **9** erzeugt werden. Verseifung (z. B. mit Natronlauge in Ethanol) ergibt dann die Dicarbonsäuren **10.** Hydrierung (z. B. mit Wasserstoff in Anwesenheit von Palladium auf Aktivkohle) ergibt die Aminodicarbonsäuren 11. Diese werden dann am Stickstoff mit einer geeigneten Schutzgruppe versehen (z. B. Sg = Benzyloxycarbonyl (Z) durch Umsetzung mit N-(Benzyloxycarbonyloxy)-succinimid in Anwesenheit von Kaliumcarbonat), wobei die Derivate **12** erhalten werden. Die Carboxylgruppen werden zu den Verbindungen **13** verestert (z. B. mit Trimethylsilyldiazomethan) und danach wird die Boc Gruppe durch Umsetzung mit einer geeigneten Säure (z. B. Trifluoressigsäure) zu den Verbindungen **14** gespalten. Zyclisierung unter basischen Bedingungen (z. B. Diisopropyl-ethyl-amin in Methanol) ergibt dann die Bizyklen **15.** Die Schutzgruppe Sg in **15** wird dann gespalten (z. B. durch Hydrierung mit Wasserstoff in Anwesenheit von Palladium auf Aktivkohle für Sg = Z) und die Amino Gruppe Boc-geschützt (z. B. mit Boc-Anhydrid), wodurch die Verbindungen **16** erhalten werden. Durch Umsetzung mit Ammoniak werden daraus die Amide **17** und **18** erhalten, welche durch Chromatographie voneinander getrennt werden können. Reaktion von **17** mit aktivierenden Reagenzien (z. B. Cyanurchlorid) ergibt anschließend die Nitrile **19,** aus denen durch Abspaltung der Boc Gruppe (z. B. mit Trifluoressigsäure) die Amine **20** erzeugt werden. Durch reduktive Aminierung der Amino Gruppe nach gängigen Verfahren werden die Verbindungen **21** erhalten.

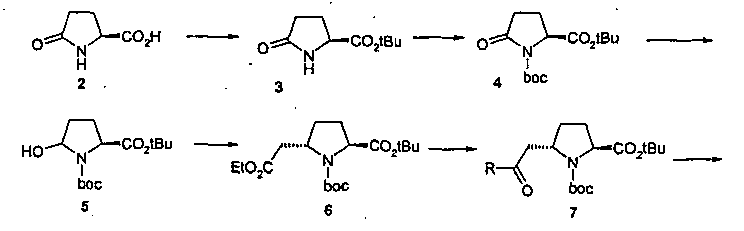

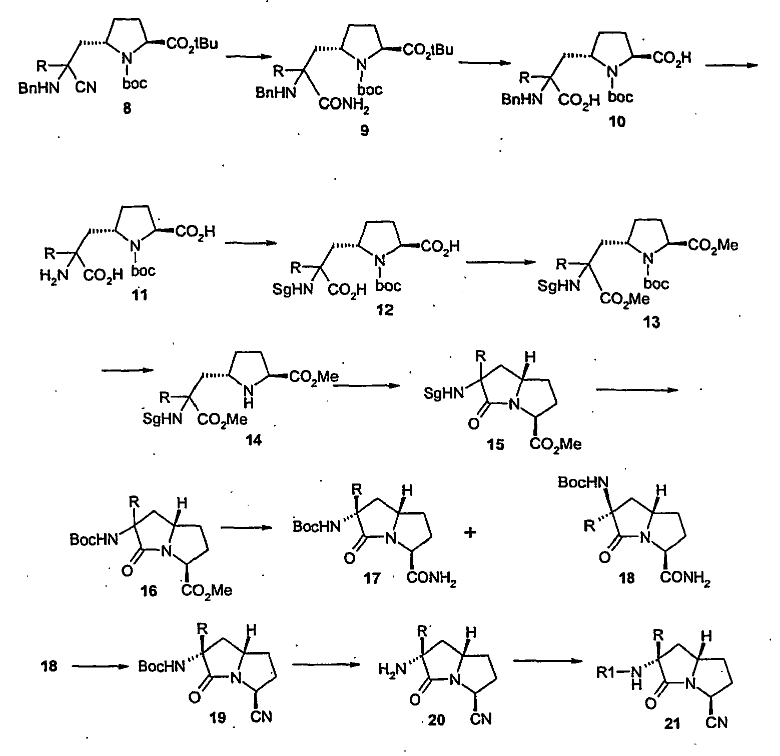

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Kilogramm Körpergewicht pro Tag, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxipropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Die Verbindung(en) der Formel (I) können auch in Kombination mit weiteren Wirkstoffen verabreicht werden.
Als weitere Wirkstoffe für die Kombinationspräparate sind geeignet:
Alle Antidiabetika, die in der Roten Liste 2003, Kapitel 12 genannt sind. Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.
Antidiabetika umfassen Insulin und Insulinderivate, wie z.B. Lantus^{®} (siehe www.lantus.com) oder HMR 1964, schnell wirkende Insuline (siehe US 6,221,633), GLP-1-Derivate wie z.B. diejenigen, die in WO 97/26265, WO 99/03861, WO 01/04156, WO 00/34331, WO00/34332, WO91/11457 und US 6,380,357 offenbart wurden, sowie oral wirksame hypoglykämische Wirkstoffe.
Die oral wirksamen hypoglykämischen Wirkstoffe umfassen vorzugsweise Sulphonylfharnstoffe, Biguanidine, Meglitinide, Oxadiazolidindione, Thiazolidindione, Glukosidase-Inhibitoren, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen wie antihyperlipidämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, oder Verbindungen wie in WO 02/50027 oder WO 04/007455 beschrieben, verabreicht.

Bei einer Ausführungsform der Erfindung werden die Verbindungen der Formel I in Kombination mit einem PPAR gamma Agonist, wie z.B. Rosiglitazon, Pioglitazon, JTT-501, Gl 262570, verabreicht.

Bei einer Ausführungsform der Erfindung werden die Verbindungen der Formel I in Kombination mit PPAR alpha Agonist, wie z.B. GW 9578, GW 7647, verabreicht.

Bei einer Ausführungsform der Erfindung werden die Verbindungen der Formel I in Kombination mit einem gemischten PPAR alpha/gamma Agonisten, wie z.B. GW 1536, AVE 8042, AVE 8134, AVE 0847, oder wie in PCT/US 11833, PCT/US 11490, DE10142734.4 beschrieben verabreicht.

Bei einer Ausführungsform der Erfindung werden die Verbindungen der Formel I in Kombination mit einem Fibrat, wie z.B. Fenofibrat, Clofibrat, Bezafibrat, verabreicht.

Bei einer Ausführungsform der Erfindung werden die Verbindungen der Formel I in Kombination mit einem MTP-Inhibitor, wie z.B. Implitapide BMS-201038, R-103757, verabreicht.

Bei einer Ausführungsform der Erfindung werden die Verbindungen der Formel I in Kombination mit Gallensäureresorptionsinhibitor (siehe z.B. US 6,245,744 oder US 6,221,897), wie z.B. HMR 1741, verabreicht.

Bei einer Ausführungsform der Erfindung werden die Verbindungen der Formel I in Kombination mit einem CETP-Inhibitor, wie z.B. JTT-705 , verabreicht.

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem LDL-Rezeptorinducer (siehe US 6,342,512), wie z.B. HMR1171, HMR1586, verabreicht.

Bei einer Ausführungsform der Erfindung werden die Verbindungen der Formel I in Kombination mit einem ACAT-Inhibitor, wie z.B. Avasimibe, verabreicht.

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.

Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen Synthetase Inhibitor, wie z.B. BMS-188494, verabreicht.

Bei einer Ausführungsform der Erfindung werden die Verbindungen der Formel I in Kombination mit einem Lipoprotein(a) antagonist, wie z.B. CI-1027 oder Nicotinsäure, verabreicht.

Bei einer Ausführungsform der Erfindung werden die Verbindungen der Formel I in Kombination mit einem Lipase Inhibitor, wie z.B. Orlistat, verabreicht.

Bei einer Ausführungsform der Erfindung werden die Verbindungen der Formel I in Kombination mit Insulin verabreicht.
Bei einer Ausführungsform werden die Verbindungen der Formel I in Kombination mit einem Sulphonylharnstoff, wie z.B. Tolbutamid, Glibenclamid, Glipizid oder Glimepirid verabreicht. Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.
Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.
Bei einer Ausführungsform werden die Verbindungen der Formel I in Kombination mit einem α-Glukosidase-Inhibitor, wie z.B. Miglitol oder Acarbose, verabreicht.

Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.
Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.:Hormone and Metabolic Research (2001), 33(9), 554-558), NPY-Antagonisten z.B. Naphthalin-1-sulfonsäure {4-[(4-amino-quinazolin-2-ylamino)-methyl]-cyclohexyhnethyl}- amid; hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo- 2,3,3a,4,6,7-hexahydro-pyrazolo[4,3-c]pyridin-5-yl)-1-(4-chloro-phenyl)-2-oxo-ethyl]- amid; (WO 01/91752)), Orexin-Antagonisten (z.B. 1-(2-Methyl-benzoxazol-6-yl)-3-[1,5]naphthyridin-4-yl-harnstoff; hydrochloride (SB-334867-A)), Cannabinoid 1 Rezeptor Antagonisten (z.B. Rimonabant oder Verbindungen, wie in WO 02/28346 beschrieben), H3-Agonisten (3-Cyclohexyl-1-(4,4-dimethyl-1,4,6,7-tetrahydroimidazo[4,5-c]pyridin-5-yl)-propan-1- on Oxalsäuresalz (WO 00 / 63208)); TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B. 1-(4-Chloro-3-methanesulfonylmethyl-phenyl)-2-[2-(2,3-dimethyl-1H-indol-6-yloxy)- ethylamino]-ethanol; hydrochloride (WO 01/83451)), MSH (Melanocyt-stimulierendes Hormon)-Agonisten, CCK-A Agonisten (z.B. {2-[4-(4-Chloro-2,5-dimethoxyphenyl)-5-(2-cyclohexyl-ethyl)-thiazol-2-ylcarbamoyl]-5,7- dimethyl-indol-1-yl}-acetic acid Trifluoressigsäuresalz (WO 99/15525)); Serotonin-Wiederaufnahme-Inhibitoren (z.B. Dexfenfluramine), gemischte Serotonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxy-1-(2-diisopropylamino-ethylcarbamoyl)-3,4-dihydro-1H-isoquinoline-2- carboxylic acid tertbutylester (WO 01/85695)), TRH-Agonisten (siehe z.B. EP 0 462 884) entkoppelnde Protein 2-oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881),
DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR-β-Agonisten verabreicht.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphetamin oder Amphetamin.
Bei einer Ausführungsform ist der weitere Wirkstoff ein Blutdrucksenker, wie z.B. ein ACE-Hemmer.
Bei einer Ausführungsform ist der weitere Wirkstoff Fenfluramin oder Dexfenfluramin.
Bei noch einer Ausführungsform ist der weitere Wirkstoff Sibutramin.
Bei einer Ausführungsform ist der weitere Wirkstoff Orlistat.
Bei einer Ausführungsform ist der weitere Wirkstoff Mazindol oder Phentermin.

Bei einer Ausführungsform ist der weitere Wirkstoff Rimonabant.

Bei einer Ausführungsform werden die Verbindungen der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties &Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungs gemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

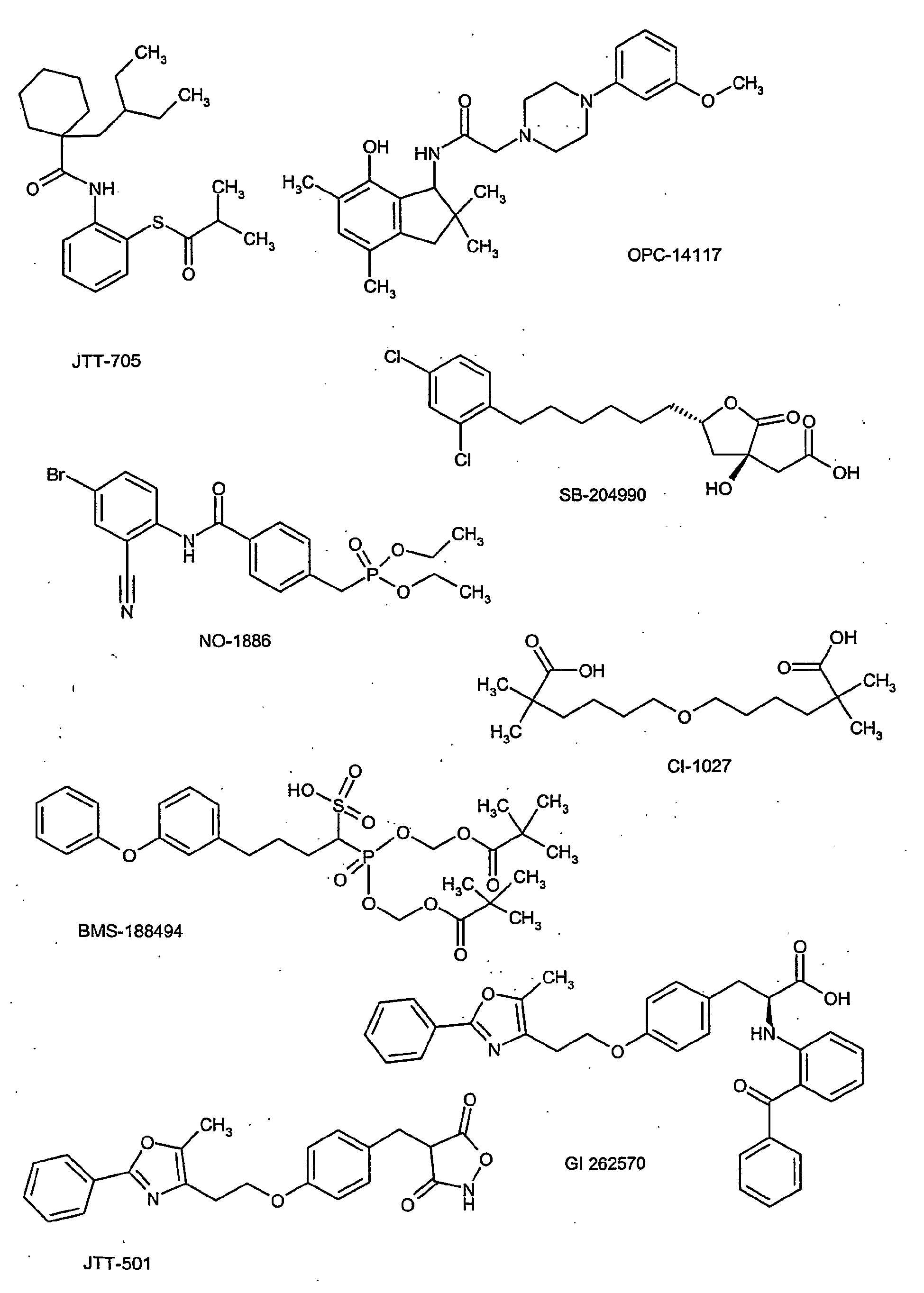

Die nachfolgend in der Tabelle I aufgeführten theoretischen Beispiele dienen zur 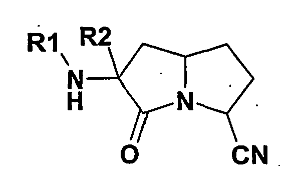 Erläuterung der Erfindung. Sie können analog zu den Ausführungsbeispielen hergestellt werden.
I

**Tabelle I**

| | **R1** | **R2** |
|---|---|---|
| a | H | Me |
| b | H | i-Pr |
| e | H | c-Hexyl |
| f | Me | H |
| g | i-Pr | H |
| I | i-Pr | Me |
| j | 1-Adamantyl | H |
| k | 1-Hydroxi-adamant-3-yl | H |
| l | Ph | H |
| m | 2-Pyridyl | H |
| n | -(CH2)2-NH-2-pyridyl | H |
| o | 1,1-Dimethyl-2-phenyl | H |
| p | 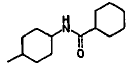 | H |
| q | 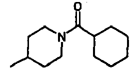 | H |
| r | Me | -CH2-CH(CH3)2 |
| t | -CH2-CH2-OH | -CH2-CH(CH3)2 |
| u | -CH2-CH2-OCH3 | 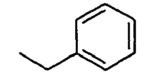 |
| v | -CH2-CH2-N(CH3)2 | -CH2-CH(CH3)2 |
| w | 2-Pyridyl | Me |
| x | 2-Thienyl | 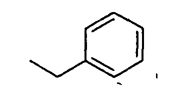 |
| y | -CH2-CH(CH3)2 | -CH2-CH(CH3)2 |
| aa | c-pentyl | CH2-CH2-CH3 |
| ba | H | CH2-CH=C(CH3)2 |
| ca | H | CH3 |
| da | H | CH2-CH3 |
| ja | H | CH2-C(CH3)3 |
| la | H | C(CH3)3 |
| ma | H | CH(CH3)(Ph) |
| pa | H | CH2-CH2-CH(CH3)2 |
| sa | H | 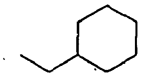 |
| ta | H | 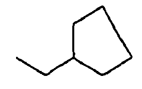 |
| va | H | 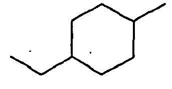 |
| wa | H | 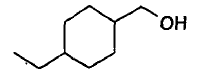 |
| xa | H | 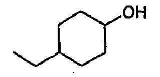 |
| ya | H | 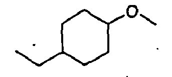 |
| za | H | 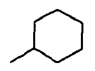 |
| ab | H | 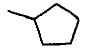 |
| bb | H | 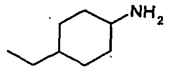 |
| eb | H | 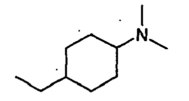 |
| gb | H | 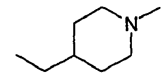 |
| pb | H | 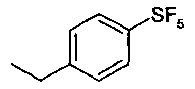 |
| qb | H | 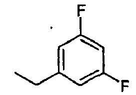 |
| rb | H | 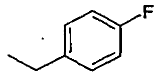 |
| sb | H | 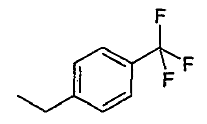 |
| tb | H | 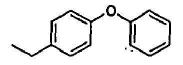 |
| ub | H | 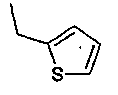 |
| vb | H | 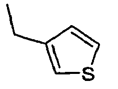 |
| wb | H | 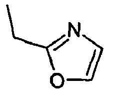 |
| zb | H | 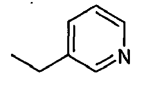 |
| ac | H | 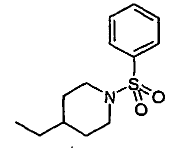 |
| bc | H | 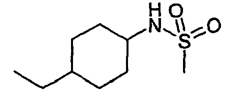 |
| cc | H | 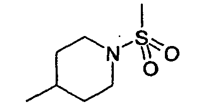 |

Die Verbindungen der Formel I zeichnen sich durch günstige Wirkungen auf den Lipid-und Kohlenhydratstoffwechsel aus, sie senken insbesondere den Blutzuckerspiegel und sind zur Behandlung von Typ 2 Diabetes, von Insulinresistenz, von Dyslipidämien und des metabolischen Syndroms / Syndrom X geeignet. Weiterhin sind die Verbindungen zur Prophylaxe und Behandlung von arteriosklerotischen Erscheinungen geeignet. Die Verbindungen können allein oder in Kombination mit weiteren Blutzucker senkenden Wirkstoffen eingesetzt werden. Die Verbindungen wirken als DPP-IV Inhibitoren und eignen sich auch zur Behandlung von Störungen des Empfindens und anderer psychiatrischen Indikationen, wie zum Beispiel Depressionen, Angstzuständen, Angstneurosen, Schizophrenie sowie zur Behandlung von Störungen assoziiert mit dem zirkadianen Rhythmus, zur Gewichtsreduktion bei Säugetieren, zur Behandlung von Immunstörungen, und zur Behandlung von Drogenmissbrauch.
Weiterhin eignen sie sich zur Behandlung von Krebs, Arthritis, Osteoarthritis, Osteoporose, Schlafstörungen, Schlaf Apnoe, weiblicher und männlicher Sexualstörungen, Entzündungen, Akne, Pigmentierung der Haut, Störungen des Steroidstoffwechsels, Hautkrankheiten, Psoriasis, Mykosen, neurodegenerativer Krankheiten, Multiple Sklerose und Alzheimer-Krankheit.

Die Wirksamkeit der Verbindungen wurde wie folgt getestet:

### Aktivitätstest

Messung der DPP-IV Aktivität:
Material:
   DPP-IV aus Schweineniere (Sigma, München)
   H-Ala-Pro-AFC (Bachem, Weil am Rhein)
Testbedingungen:
   DPP-IV (1 mU/ml, Endkonzentratio)
   H-Ala-Pro-AFC (15µM Endkonzentration)
      in Tris/HCl (40 mM, pH 7.4), Gesamtvolumen 0,2 ml

Die Reaktion wurde bei Raumtemperatur für unterschiedliche Zeiträume (typischerweise 10 min) durchgeführt und am Ende der Reaktion durch Zugabe von 20µl ZnCl₂ (1 M) gestoppt. Der Umsatz von H-Ala-Pro-AFCwurde fluorimetrisch durch Messung der Emission bei 535nm nach Anregung bei 405 nm bestimmt. Im Falle der Zugabe von Inhibitoren wurde das zugegebene Puffervolumen so angepasst, dass ein Gesamtvolumen der Testmischung von 200µl eingehalten wurde.
IC50 Werte für Inhibitoren wurden durch Variation der Inhibitorkonzentrationen bei der angegebenen Substratkonzentration von 15µM bestimmt. Ki und Km Werte wurden durch enstsprechende Variation von Substrat- und Inhibitorkonzentration wie beschrieben (Dixon, M. and Webb, E.C.(1979) Enzymes , third edition, pp. 47-206, Academic Press) ermittelt. Die Werte für Km, IC50 and Ki wurden mit einem kommerziell erhältlichen Software-Paket errechnet ( Leatherbarrow, R.J. (1992) GraFit Version 3.0, Erithacus Software Ltd. Staines, U.K.).

Die Messung ergab folgenden Wert:

| Beispiel Nr. | IC-50 | Bemerkungen |
|---|---|---|
| 1) | | |

Das Ausführungsbeispiel 1 wurde wie folgt hergestellt:

### 1) (3S,6R,7aS)-6-Amino-5-oxo-hexahydro-pyrrolizine-3-carbonitrile-trifluoracetat

### la) (S)-5-Oxo-pyrrolidin-2-carbonsäure-tert-butylester

129 g L-Pyroglutaminsäure wurden in 21 tert.-Butylacetat suspendiert. Dazu wurden 30 ml einer 70 %-igen Lösung von Perchlorsäure in wasser gegeben. Anschließend ließ man 12 h bei Raumtemperatur rühren. Man kühlte auf 0°C ab und stellte den pH-Wert der Lösung durch vorsichtige Zugabe einer 2 N NaOH Lösung auf 7 ein. Die Phasen wurden getrennt und die organische Phase 2 mal mit je 300 ml einer gesättigten Natriumchlorid Lösung gewaschen. Danach wurde die organische Phase mit Natriumsulfat getrocknet. Die Lösungsmittel wurden im Vakuum entfernt und der Rückstand mit Heptan verrührt. Der so erhaltene Feststoff wurde abgesaugt. Die Mutterlauge wurde im Vakuum eingeengt und auch der so erhaltene Feststoff abgesaugt.
Ausbeute: 157 g (S)-5-Oxo-pyrrolidin-2-carbonsäure-tert-butylester
MS: 186 (M+H)

### 1b) (S)-S-Oxo-pyrrolidin-1,2-dicarbonsäure-di-tert-butylester

120 g (S)-5-Oxo-pyrrolidin-2-carbonsäure-tert-butylester wurden in 1,31 Dichlormethan gelöst und auf 0°C gekühlt. Dazu wurden 3,9 g 4-Dimethylaminopyridin sowie 141 g Di-tert.-Butyl-dicarbonat gegeben. Man ließ 1 h bei 0°C und 12 h bei Raumtemperatur rühren. Danach wurde das Volumen der Lösung auf ca. 300 ml im Vakuum eingeengt. Die so erhaltene Lösung wurde über 500 g Kieselgel filtriert, wobei zunächst mit Dichlormethan und danach mit 5 % Essigester in Dichlormethan eluiert wurde. Die Produktfraktionen wurden vereinigt und im Vakuum von den Lösungsmitteln befreit.
Ausbeute: 146 g (S)-5-Oxo-pyrrolidin-1,2-dicarbonsäure-di-tert-butylester
MS: 186 (M+H-Boc)

### 1c) (S)-5-Hydroxy-pyrrolidin-1,2-dicarbonsäure-di-tert-butylester

93 g (S)-5-Oxo-pxrolidin-1,2-dicarbonsäure-di-tert-butylester wurden in 600 ml THF gelöst und auf -78°C gekühlt. Man tropfte 392 ml einer 1 N Lösung von Lithiumtriethylborhydrid in THF innerhalb von 90 Min. zu. Danach wurde 30 Min. bei -70°C nachgerührt. Anschließend wurden 240 ml gesättigte Natriumhydrogencarbonat Lösung zu der Lösung getropft. Man ließ die Temperatut auf-15°C kommen und tropfte dann 160 ml einer 30 %-igen Wasserstoffperoxid Lösung zu. Danach ließ man 30 Min. bei Raumtemperatur nachrühren. Die Phasen wurden dann getrennt und die organische Phase im Vakuum eingeengt. Der Rückstand wurde in 500 ml tert.-Butylmethylether aufgenommen, 3 mal mit je 300 ml einer 10 %-igen Lösung von Natriumhydrogencarbonat in Wasser und 2 mal mit je 300 ml einer gesättigten Natriumchlorid Lösung gewaschen. Die organische Phase wurde mit Natriumsulfat getrocknet und die Lösungsmittel im Vakuum entfernt. Das so erhaltene Rohprodukt wurde ohne weitere Reinigung in der nächsten Umsetzung eingesetzt.
Ausbeute: 76 g (S)-5-Hydroxy-pyuolidin-1,2-dicarbonsäure-di-tert-butylester
MS: 170 (M+H-Boc-H2O)

### ld) (2S,5S)-5-Ethoxycarbonylmethyl-pyrrolidin-1,2-dicarbonsäure-di-tert-butylester

3,2 g einer 60 %-igen Diespersion von Natriumhydrid in Mineralöl wurden in 300 ml THF suspendiert. Dazu wurden 20,7 g Diethylphosphonoessigsäureethylester in 50 ml THF getropft. Man ließ 2 h bei Raumtemperatur nachrühren und kühlte dann auf-78°C. Danach tropfte man eine Lösung von 19 g (S)-5-Hydroxy-pyrrolidin-1,2-dicarbonsäure-di-tert-butylester in 100 ml THF zu, ließ auf Raumtemperatur kommen und 24 h rühren. Das THF wurde im Vakuum entfernt und der Rückstand zwischen 250 ml Wasser und 250 ml Dichlormethan verteilt. Die organische Phase wurde mit Natriumsulfat getrocknet und im Vakuum eingeengt. Der Rückstand wurde an Kieselgel chromatographiert. Das Produkt wurde als ca. 95:5 Diastereomerengemisch erhalten (ca. 95 % 5S, 5 % 5R).
Ausbeute: 16 g (2S,SS)-5-Ethoxycarbonylmethyl-pyrrolidin-1,2-dicarbonsäure-di-tert-butylester (enthält ca. 5 % (2S,5R)-5-Ethoxycarbonylmethyl-pyrrolidin-1,2-dicarbonsäure-di-tert-butylester)
MS: 202 (M+H-Boc-tert.-Butyl)

### le) (2S,5S)-5-Hydroxyethyl-pyrrolidin-1,2-diearbonsäure-di-tert-butylester

43 g des Produktes aus 1 d wurden in 300 ml Diethylether gelöst. Dazu tropfte man eine Lösung von 3,1 g Lithiumborhydrid in 300 ml Diethylether. Man ließ 6 h nachrühren und beendete die Reaktion durch vorsichtige Zugabe von 300 ml einer gesättigten Kaliumhydrogencarbonat Lösung. Die Phasen wurden getrennt und die organische Phase mit 250 ml einer gesättigten Natriumchlorid Lösung gewaschen. Die organische Phase wurde mit Natriumsulfat getrocknet und das Lösungsmittel im Vakuum entfernt. Der Rückstand wurde an Kieselgel chromatographiert.
Ausbeute 33 g (2S,5S)-5-Hydroxyethyl-pyrrolidin-1,2-dicarbonsäure-di-tert-butylester
MS: 204 (M+H-Boc-tert.-Butyl)

### 1f) (2S,5S)-5-(2-Benzylamino-2-eyano-ethyl)-pyrrolidin-1,2-dicarbonsäure-di-tert-butylester

1,4 g (2S,SS)-5-Hydroxyethyl-pyrrolidin-1,2-dicarbonsäure-di-tert-butylester wurden in 10 ml Dichlormethan gelöst. Dazu tropfte man eine Lösung von 2,45 g 1,1-Dihydro-1,1,1-triacetoxy-1,2-benzjodoxol-3(1H)-on (Dess-Martin-Periodinan) in 25 ml Dichlormethan. Man ließ 30 Min. nachrühren und verdünnte dann mit 200 ml tert.-Butylmethylether. Es wurde 1 mal mit 150 ml einer 10 %-igen Natriumhydrogencarbonat Lösung, 1 mal mit 150 ml einer 5 %-igen Natriumthiosulfat Lösung und 1 mal mit 150 ml einer gesättigten Natriumchlorid Lösung gewaschen. Die organische Phase wurde mit Natriumsulfat getrocknet und die Lösungsmittel im Vakuum entfernt. Das so erhaltene Rohprodukt wurde in 20 ml THF gelöst. Dazu wurden 535 mg Benzylamin gegeben und für 1 h nachgerührt. Danach gab man nacheinander 0,66 ml Trimethylsilylcyanid und 110 mg Indium-III-chlorid. Man ließ 14 h bei Raumtemperatur rühren. Nach Filtration über Kieselgur wurden die Lösungsmittel im Vakuum entfernt und der Rückstand an Kieselgel chromatographiert.
Ausbeute: 1,15 g (2S,SS)-5-(2-Benzylamino-2-cyano-ethyl)-pyrrolidin-1,2-dicarbonsäure-di-tert-butylester
MS: 430 (M+H)

### lg) (2S,5S)-S-(2-Benzylamino-2-carbamoyl-ethyl)-pyrrolidin-1,2-dicarbonsäure-di-tert-butylester

2,6 g (2S,5S)-S-(2-Benzylamino-2-cyano-ethyl)-pyrrolidin-1,2-dicarbonsäure-di-tert-butylester wurden in 6.5 ml Dimethylsulfoxid gelöst. Dazu gab man 373 mg feingemahlenes Kaliumcarbonat und 1,08 ml einer 30 %-igen Wasserstoffperoxid Lösung. Man ließ 14 h bei Raumtemperatur rühren und erwärmte danach 30 Min. auf 35°C. Die Lösung wurde mit 60 ml Wasser verdünnt und 3 mal mit je 100 ml Essigester extrahiert. Die vereinigten organischen Phasen wurden 1 mal mit 120 ml einer gesättigten Natriumchlorid Lösung gewaschen und mit Natriumsulfat getrocknet. Die Lösungsmittel wurden im Vakuum entfernt und der Rückstand an Kieselgel chromatographiert.
Ausbeute: 2,4 g (2S,5S)-5-(2-Benzylamino-2-carbamoyl-ethyl)-pyrrolidin-1,2-dicarbonsäure-di-tert-butylester
MS: 448 (M+H)

### 1h) (2S,5S)-5-(2-Benzylamino-2-carboxy-ethyl)-pyrrolidin-1,2-dicarbonsäure-1-tert-butylester

2,2 g (2S,5S)-5-(2-Benzylamino-2-carbamoyl-ethyl)-pyrrolidin-1,2-dicarbonsäure-di-tert-butylester wurden in 20 ml Ethanol und 5 ml Wasser gelöst. Dazu gab man 1,2 g festes Natriumhydroxid . Anschließend wurde die Lösung 36 h am Rückfluß gekocht. Zur Aufarbeitung kühlte man auf Raumtemperatur , neutralisierte mit Essigsäure und entfernte das Ethanol im Vakuum.. Der Rückstand wurde mit 20 ml Wasser verdünnt und mit essigsäure ein pH-Wert von 3 eingestellt. Es wurde 3 mal mit je 30 ml Essigester/Butanol 5:1 extrahiert. Die organischen Phasen wurden vereinigt und im Vakuum eingeengt. Der Rückstand wurde an Kieselgel chromatographiert.
Ausbeute:
1700 mg (2S,5S)-5-(2-Benzylamino-2-carboxy-ethyl)-pyrrolidin-1,2-dicarbonsäure-1-tert-butylester
MS: 393 (M+H)

### li) (25,5S)-5-(2-Amino-2-carboxy-ethyl)-pyrrolidin-1,2-dicarbonsäure-1-tert-butylester Hydrogenacetat

1600 mg (2S,5S)-5-(2-Benzylamino-2-carboxy-ethyl)-pyrrolidin-1,2-dicarbonsäure-1-tert-butylester wurden in 30 ml Essigsäure gelöst. Man gab 100 mg 20 % Palladiumhydroxid auf Aktivkohle hinzu und hydrierte für 1 h unter 1 Bar Wasserstoffatmosphäre. Danach wurde 10 Min. Ar über die Lösung geleitet. Der Katalysator wurde über Kieselgur abfiltriert und der Filterkuchen mit 20 ml Essigsäure gewaschen. Die vereinigten organische Phasen wurde im Vakuum eingeengt. Das Rohprodukt wurde ohne weitere Reinigung in der nächsten Umsetzung eingesetzt.
Ausbeute: 1500 mg (2S,5S)-5-(2-Amino-2-carboxy-ethyl)-pyrrolidin-1,2-dicarbonsäure-1-tert-butylester Hydrogenacetat
MS: 303 (M+H)

### 1j) (2S,5S)-5-(2-Benzyloxycarbonylamino-2-carboxy-ethyl)-pyrrolidin-1,2-dicarbonsäure-1-tert-butylester

1450 mg (2S,5S)-5-(2-Amino-2-carboxy-ethyl)-pyrrolidin-1,2-dicarbonsäure-1-tert-butylester Hydrogenacet wurden in 30 ml Wasser und 40 ml Dioxan gelöst. Anschließend gab man nacheinander 1600 mg Kaliumhydrogencarbonat und 997 mg N-(Benzyloxycarbonyloxy)-succinimid zu. Man ließ 4 h bei Raumtemperatur rühren und entfernte dann das Dioxan im Vakuum. Der Rückstand wurde mit 20 ml Wasser verdünnt und der pH-Wert mit 1 N HCl Lösung auf 2 eingestellt. Die wäßr. Phase wurde 4 mal mit je 60 ml Ethylacetat extrahiert. Die vereinigten organischen Phasen wurden mit Natriumsulfat getrocknet und im Vakuum eingeengt.
Ausbeute: 1570 mg (2S,5S)-5-(2-Benzyloxycarbonylamino-2-carboxy-ethyl)-pyrrolidin-1,2-dicarbonsäure-1-tert-butylester
MS: 337 (M+H-Boc)

### lk) (2S,5S)-5-(2-Benzyloxycarbonylamino-2-methoxycarbonyl-ethyl)-pyuolidin-1,2-dicarbonsäure-1-fert-butylester-2-methylester

1450 mg (2S,5S)-5-(2-Benzyloxycarbonylamino-2-carboxy-ethyl)-pyrrolidin-1,2-dicarbonsäure-1-tert-butylester wurden in 15 ml THF und 5 ml Methanol gelöst. Man kühlte auf 0°C ab und gab 4 ml einer 2 M Lösung von Trimethylsilyldiazomethan in Hexan zu. Innerhalb von 1 h ließ man auf Raumtemperatur kommen und beendete dann die Reaktion durch vorsichtige Zugabe von Essigsäure. Man ließ 10 Min. nachrühren und entfernte dann die Lösungsmittel im Vakuum. Der Rückstand wurde durch Chromatographie an Kieselgel gereinigt .
Ausbeute: 1500 mg (2S,5S)-5-(2-Benzyloxycarbonylamino-2-methoxycarbonyl-ethyl)-pyrrolidin-1,2-dicarbonsäure-1-tert-butylester-2-methylester
MS: 365 (M+H-Boc)

### 11) (35,7aS)-6-Benzyloxycarbonylamino-5-oxo-hexahydro-pyrrolizin-3-carbonsäure-methylester

1500 mg (2S,5S)-5-(2-Benzyloxycarbonylamino-2-methoxycarbonyl-ethyl)-pyrrolidin-1,2-dicarbonsäure-1-tert-butylester-2-methylester wurden mit 0,2 ml Thioanisol versetzt. Anschließend gab man 10 ml Trifluoressigsäure zu und ließ 45 min. rühren. Danach wurden die Lösungsmittel im Vakuum entfernt und der Rückstand in 10 ml Pyridin aufgenommen. Man gab 1,1 ml Diisopropyl-ethyl-amin zu und erhitzte für 3h auf 95 °C. Die Lösungsmittel wurden im Vakuum entfernt und der Rückstand an Kieselgel chromatographiert.
Ausbeute: 540 mg (3S,7aS)-6-B-enzyloxycarbonylamino-5-oxo-hexahydro-pyrrolizin-3-carbonsäure-methylester
MS: 333 (M+H)

### 111) (3S,7aS)-6-tert-Butoxycarbonylamino-5-oxo-hexahydro-pyrrolizin-3-carbonsäuremethylester

540 mg (3S,7aS)-6-Benzyloxycarbonylamino-5-oxo-hexahydro-pyrrolizin-3-carbonsäuremethylester wurden in 10 ml Eisessig gelöst. Man gab 50 mg 10 % Palladium auf Aktivkohle hinzu und hydrierte für 1 h unter 1 Bar Wasserstoffatmosphäre. Anschließend leitete man für 10 Min. Ar über die Lösung und filtrierte dann über einen Membranfilter. Das Lösungsmittel wurde im Vakuum entfernt und der Rückstand in 30 ml Acetonitril aufgenommen. Man gab nacheinander 350 mg Di-tert.-butyl-dicarbonat und 0,28 ml Diisopropylethylamin hinzu und ließ 1 h bei Raumtemperatur rühren. Die Lösungsmittel wurden im Vakuum entfernt und der Rückstand an Kieselgel chromatographisch gereinigt.
Ausbeute: 440 mg (3S,7aS)-6-tert-Butoxycarbonylamino-5-oxo-hexahydro-pyrrolizin-3-carbonsäure-methylester
MS: 243 (M+H-tBu)

### 1m) ((2R,5S,7aS)-5-Carbamoyl-3-oxo-hexahydro-pyrrolizin-2-yl)-carbaminsäure-tert-butylester und ((2S,5S,7aS)-5-Carbamoyl-3-oxo-hexahydro-pyrrolizin-2-yl)-carbambsäure-tert-butylester

465 mg (3S,7aS)-6-tert-Butoxycarbonylamino-5-oxo-hexahydro-pyrrolizin-3-carbonsäure-methylester wurden bei 0°C mit 100 ml einer 7M Lösung von Ammoniak in Methanol versetzt. Man ließ langsam auf Raumtemperatur kommen und 14 h nachrühren. Danach wurden die Lösungsmittel im Vakuum entfernt. Der Rückstand wurde an Kieselgel chromatographiert. Ausbeute: 200 mg ((2R,5S,7aS)-5-Carbamoyl-3-oxo-hexahydro-pyrrolizin-2-yl)-carbaminsäure-tert-butylester
MS: 228 (M+H-tBu)
Und 170 mg ((2S,5S,7aS)-5-Carbamoyl-3-oxo-hexahydro-pyrrolizin-2-yl)-carbaminsäure-tert-butylester
MS: 228 (M+H-tBu)

### In) ((2R,5S,7aS)-5-Cyano-3-oxo-hexahydro-pyrrolizin-2-yl)-carbaminsäure-tert-butylester

100 mg ((2R,5S,7aS)-5-Carbamoyl-3-oxo-hexahydro-pyrrolizin-2-yl)-carbaminsäure-tert butylester wurden in 2 ml Dimethylformamid gelöst und mit 65 mg Cyanurchlorid versetzt. Man ließ 3 h rühren und entfernte das Dimethylformamid im Vakuum. Der Rückstand wurde an Kieselgel chromatographiert.
Ausbeute: 85 mg ((2R,5S,7aS)-5-Cyano-3-oxo-hexahydro-pyrrolizin-2-yl)-carbaminsäure-tert-butylester
MS: 210 (M+H-tBu)

### lo) (3S,6R,7aS)-6-Amino-S-oxo-hexahydro-pyrrolizin-3-carbonitrile-trifluoracetat

80 mg ((2R,5S,7aS)-5-Cyano-3-oxo-hexahydro-pyrrolizin-2-yl)-carbaminsäure-tert-butylester wurden für 30 Min. mit 10 % Thioanisol in 1 ml Trifluoressigsäure zur Reaktion gebracht. Danach wurden die Lösungsmittel im Vakuum entfernt und der Rückstand aus Diisopropylether ausgerührt.
Ausbeute: 73 mg (3S,6R,7aS)-6-Amino-5-oxo-hexahydro-pyrrolizin-3-carbonitrile-trifluoracetat
MS: 166 (M+H)

## Patentansprüche

1. Verbindungen der Formel I, 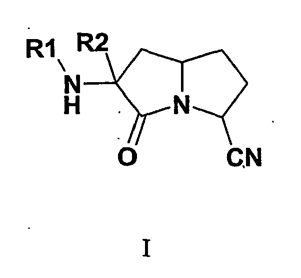 worin bedeuten
R1 H, (C₁-C₁₀)-Alkyl, (C₃-C₁₀)-Cycloalkyl, (C₂-C₆)-Alkenyl, (C₂-C₆)-Alkinyl, (C₆- C₁₀)-Aryl, Heterozyklyl, COR3, COOR3, CONR3R4, CN, wobei die Alkyl-, Cycloalkyl-, Alkenyl-, Alkinyl-, Aryl- und Heterozyklyl Reste ein-oder mehrfach substituiert sein können mit F, Cl, Br, I, CN, NO₂, SH, SF₅, OH, (C₁- C₆)-Alkyl, -CF₃, (C₂-C₆)-Alkenyl, (C₂-C₆)-Alkmyl, OR3, OP(O)(OR3)₂, NR3R4, NR3CONR3R4_{;} COR3, OCOR3, OCOOR3, COOR3, CONR3R4, OCONR3R4, (C₁-C₆)-Alkylen-OR3, (C₁-C₆)-Alkylen-NR3R4, (C₁-C₆)-Alkylen-NR3SO₂R4, (C₁-C₆)-Alkylen-SR3, Alkylen-S(O)R3, Alkylen-S(O)₂R3, Alkylen- S(O)₂NR3R4, (C₁-C₆)-Alkylen-COR3, (C₁-C₆)-Alkylen-COOR3, (C₁-C₆)- Alkylen-CONR3R4, SR3, SOR3, SO₂R3, SO₂NR3R4, NR3SO₂R4, (C₁-C₆)- Alkylen-(C₃-C₁₀)-Cycloalkyl, (C₁-C₆)-Alkylen-(C₆-C₁₀)-Aryl, (C₁-C₆)-Alkylen- Heterozyklyl, (C₃-C₁₀)-Cycloalkyl, (C₆-C₁₀)-Aryl oder Heterozyklyl;
R2 H, (C₁-C₁₀)-Alkyl, (C₃-C₁₀)-Cycloalkyl, (C₂-C₆)-Alkenyl, (C₂-C₆)-Alkinyl, (C₆- C₁₀)-Aryl, Heterozyklyl, COR3, COOR3, CONR3R4, CN, wobei die Alkyl-, Cycloalkyl-, Alkenyl-, Alkinyl-, Aryl- und Heterozyklyl Reste ein-oder mehrfach substituiert sein können mit F, Cl, Br, I, CN, NO₂, SH, SF₅, OH, (C₁- C₆)-Alkyl, -CF₃, (C₂-C₆)-Alkenyl, (C₂-C₆)-Alkmyl, OR3, OP(O)(OR3)₂, NR3R4, NR3CONR3R4, COR3, OCOR3, OCOOR3, COOR3, CONR3R4, OCONR3R4, (C₁-C₆)-Alkylen-OR3, (C₁-C₆)-Alkylen-NR3R4, (C₁-C₆)-Alkylen-NR3SO₂R4, (C₁-C₆)-Alkylen-SR3, Alkylen-S(O)R3, Alkylen-S(O)₂R3, Alkylen- S(O)₂NR3R4, (C₁-C₆)-Alkylen-COR3, (C₁-C₆)-Alkylen-COOR3, (C₁-C₆)- Alkylen-CONR3R4, SR3, SOR3, SO₂R3, SO₂NR3R4, NR3SO₂R4, (C₁-C₆)- Alkylen-(C₃-C₁₀)-Cyloalkyl, (C₁-C₆)-Alkylen-(C₆-C₁₀)-Aryl, (C₁-C₆)-Alkylen- Heterozyklyl, (C₃-C₁₀)-Cycloalkyl, (C₆-C₁₀)-Aryl oder Heterozyklyl;
R3, R4 unabhängig voneinander H, (C₁-C₆)-Alkyl, -CF₃, (C₃-C₁₀)-Cycloalkyl, (C₆-C₁₀)- Aryl, Heterozyklyl, (C₁-C₆)-Alkylen-CONR5R6, CONR5R6, (C₁-C₆)-Alkylen- COOR5, COOR5, COR5, (C₁-C₆)-Alkylen-COR5, (C₁-C₆)-Alkylen-OR5, (C₁- C₆)-Alkylen-NR5R6; (C₁-C₆)-Alkylen-SR5, (C₁-C₆)-Alkylen-S(O)R5, (C₁-C₆)- Alkylen-S(O)₂R5, S(O)R5, S(O)₂R5, (C₁-C₄)-Alkylen-(C₆-C₁₀)-Aryl oder (C₁- C₄)-Alkylen-Heterozyklyl;
R5, R6 unabhängig voneinander H, (C₁-C₆)-Alkyl, (C₁-C₆)-Alkylen-(C₆-C₁₀)-Aryl, -(C₆- C₁₀)-Aryl, Heterozyklyl, (C₁-C₆)-Alkylen-Heterozyklyl;
sowie deren physiologisch verträglichen Salze.

2. Verbindungen der Formel I, gemäß Anspruch 1, **dadurch gekennzeichnet, dass** darin bedeuten
R1 H
R2 H, (C₁-C₁₀)-Alkyl, (C₃-C₁₀)-Cycloalkyl, (C₂-C₆)-Alkenyl, (C₂-C₆)-Allcinyl, (C₆- C₁₀)-Aryl, Heterozyklyl, COR3, COOR3, CONR3R4, CN, wobei die Alkyl-, Cycloalkyl-, Alkenyl-, Alkinyl-, Aryl- und Heterozyklyl Reste ein-oder mehrfach substituiert sein können mit F, Cl, Br, I, CN, NO₂, SH, SF₅, OH, (C₁- C₆)-Alkyl, -CF₃, (C₂-C₆)-Alkenyl, (C₂-C₆)-Alkinyl, OR3, OP(O)(OR3)₂, NR3R4, NR3CONR3R4, COR3, OCOR3, OCOOR3, COOR3, CONR3R4, OCONR3R4, (C₁-C₆)-Alkylen-OR3, (C₁-C₆)-Alkylen-NR3R4, (C₁-C₆)-Akylen-NR3SO₂R4, (C₁-C₆)-Alkylen-SR3, Alkylen-S(O)R3, Alkylen-S(O)₂R3, Alkylen- S(O)₂NR3R4, (C₁-C₆)-Alkylen-COR3, (C₁-C₆)-Alkylen-COOR3, (C₁-C₆)- Alkylen-CONR3R4, SR3, SOR3, SO₂R3, SO₂NR3R4, NR3SO₂R4, (C₁-C₆)- Alkylen-(C₃-C₁₀)-Cycloalkyl, (C₁-C₆)-Alkylen-(C₆-C₁₀)-Aryl, (C₁-C₆)-Alkylen- Heterozyklyl, (C₃-C₁₀)-Cycloalkyl, (C₆-C₁₀)-Aryl oder Heterozyklyl;
R3, R4 unabhängig voneinander H, (C₁-C₆)-Alkyl, -CF₃, (C₃-C₁₀)-Cycloalkyl, (C₆-C₁₀)- Aryl, Heterozyklyl, (C₁-C₆)-Alkylen-CONR5R6, CONR5R6, (C₁-C₆)-Alkylen- COOR5, COOR5, COR5, (C₁-C₆)-Alkylen-COR5, (C₁-C₆)-Alkylen-OR5, (C₁- C₆)-Alkylen-NR5R6, (C₁-C₆)-Alkylen-SR5, (C₁-C₆)-Akylen-S(O)R5, (C₁-C₆)- Alkylen-S(O)₂R5, S(O)R5, S(O)₂R5, (C₁-C₄)-Alkylen-(C₆-C₁₀)-Aryl oder (C₁- C₄)-Alklen-Heterozyklyl;
R5, R6 unabhängig voneinander H, (C₁-C₆)-Alkyl, (C₁-C₆)-Alkylen-(C₆-C₁₀)-Aryl, -(C₆- C₁₀)-Aryl, Heterozyklyl, (C₁-C₆)-Alkylen-Heterozyklyl;
sowie deren physiologisch verträglichen Salze.

3. Verbindungen der Formel I, gemäß Anspruch 1 oder 2, **dadurch gekennzeichnet, dass** darin bedeuten
R1 H
R2 (C₁-C₁₀)-Alkyl, (C₃-C₁₀)-Cycloalkyl, (C₂-C₆)-Alkenyl, (C₂-C₆)-Alkinyl, (C₆-C₁₀)- Aryl, Heterozyklyl, COR3, COOR3, CONR3R4, CN, wobei die Alkyl-, Cycloalkyl-, Alkenyl-, Alkinyl-, Aryl- und Heterozyklyl Reste ein-oder mehrfach substituiert sein können mit F, Cl, Br, I, CN, NO₂, SH, SF₅, OH, (C₁- C₆)-Alkyl, -CF₃, (C₂-C₆)-Alkenyl, (C₂-C₆)-Alkinyl, OR3, OP(O)(OP3)₂, NR3R4, NR3CONR3R4, COR3, OCOR3, OCOOR3, COOR3, CONR3R4, OCONR3R4, (C₁-C₆)-Alkylen-OR3, (C₁-C₆)-Alkylen-NR3R4, (C₁-C₆)-Alkylen-NR3SO₂R4, (C₁-C₆)-Alkylen-SR3, Alkylen-S(O)R3, Alkylen-S(O)₂R3, Alkylen- S(O)₂NR3R4, (C₁-C₆)-Alkylen-COR3, (C₁-C₆)-Alkylen-COOR3, (C₁-C₆)- Alkylen-CONR3R4, SR3, SOR3, SO₂R3, SO₂NR3R4, NR3SO₂R4, (C₁-C₆)- Alkylen-(C₃-C₁₀)-Cycloalkyl, (C₁-C₆)-Alkylen-(C₆-C₁₀)-Aryl, (C₁-C₆)-Alkylen- Heterozyklyl, (C₃-C₁₀)-Cycloalkyl, (C₆-C₁₀)-Aryl oder Heterozyklyl;
R3, R4 unabhängig voneinander H, (C₁-C₆)-Alkyl, -CF₃, (C₃-C₁₀)-Cycloalkyl, (C₆-C₁₀)- Aryl, Heterozyklyl, (C₁-C₆)-Alkylen-CONR5R6, CONR5R6, (C₁-C₆)-Alkylen- COOR5, COOR5, COR5, (C₁-C₆)-Alkylen-COR5, (C₁-C₆)-Alkylen-OR5, (C₁- C₆)-Alkylen-NR5R6, (C₁-C₆)-Alkylen-SR5, (C₁-C₆)-Alkylen-S(O)R5, (C₁-C₆)- Alkylen-S(O)₂R5, S(O)R5, S(O)₂R5, (C₁-C₄)-Alkylen-(C₆-C₁₀)-Aryl oder (C₁- C₄)-Alkylen-Heterozyklyl;
R5, R6 unabhängig voneinander H, (C₁-C₆)-Alkyl, (C₁-C₆)-Alkylen-(C₆-C₁₀)-Aryl, -(C₆- C₁₀)-Aryl, Heterozyklyl, (C₁-C₆)-Alkylen-Heterozyklyl;
sowie deren physiologisch verträglichen Salze.

4. Verbindungen der Formel I, gemäß einem oder mehreren der Ansprüche 1 bis 3, **dadurch gekennzeichnet, dass** darin bedeuten
R1 H;
R2 (C₁-C₁₀)-Alkyl, (C₃-C₁₀)-Cycloalkyl, (C₂-C₆)-Alkenyl, (C₂-C₆)-Alkinyl, (C₆-C₁₀)- Aryl, ein Pyrrolidino-, Piperidino-, Hexamethylenimino-, Morpholino-, Piperazino-, Thiomorpholino- oder Homopiperazino-Rest; wobei die Alkyl-, Cycloalkyl-, Alkenyl-, Alkinyl-, Pyrrolidino-, Piperidino-, Hexamethylenimino-, Morpholino-, Piperazino-, Thiomorpholino-, Homopiperazino- und Aryl-Reste ein-oder mehrfach substituiert sein können mit F, Cl, Br, CN, SF₅, OH, (C₁-C₆)- Alkyl, -CF₃, (C₂-C₆)-Alkenyl, OR3, NR3R4, NR3CONR3R4, COR3, OCOR3, CO₂R3, CONR3R4, OCONR3R4, (C₁-C₆)-Alkylen-OR3, (C₁-C₆)-Alkylen- NR3R4, (C₁-C₆)-Alkylen-NR3SO₂R4, (C₁-C₆)-Alkylen-SR3, Alkylen-S(O)R3, Alkylen-S(O)₂R3, Alkylen-S(O)₂NR3R4, (C₁-C₆)-Alkylen-COR3, (C₁-C₆)- Alkylen-CO₂R3, (C₁-C₆)-Alkylen-CONR3R4, SR3, SOR3, SO₂R3, SO₂NR3R4, NR3SO₂R4, (C₁-C₆)-Alkylen-(C₃-C₁₀)-Cycloalkyl, (C₁-C₆)-Alkylen-(C₆-C₁₀)- Aryl, (C₁-C₆)-Alkylen-Heterozyklyl, (C₃-C₁₀)-Cycloalkyl, (C₆-C₁₀)-Aryl, das mit F, Cl, Br, I, CN, OH, -CF₃, (C₁-C₆)-Alkyl, OR3, NR3R4, COR3, CO2R3, CONR3R4 ein oder mehrfach substituiert sein kann, oder Heterozyklyl, das mit F, Cl, Br, CN, NO₂, OH, -CF₃, (C₁-C₆)-Alkyl, OR3, NR3R4, COR3, CO2R3, CONR3R4 ein oder mehrfach substituiert sein kann;
R3, R4 unabhängig voneinander H, (C₁-C₆)-Alkyl, -CF₃, (C₃-C₁₀)-Cycloalkyl, (C₆-C₁₀)- Aryl, Heterozyklyl, (C₁-C₆)-Alkylen-CONR5R6, (C₁-C₆)-Alkylen-COOR5, (C₁- C₆)-Alkylen-COR5, (C₁-C₆)-Alkylen-OR5, (C₁-C₆)-Alkylen-NR5R6, (C1-C₆)- Alkylen-SR5, (C₁-C₆)-Alkylen-S(O)R5, (C₁-C₆)-Alkylen-S(O)₂R5, (C₁-C₄)- Alkylen-(C₆-C₁₀)-Aryl oder (C₁-C₄)-Alkylen-Heterozyklyl;
R5, R6 unabhängig voneinander H, (C₁-C₆)-Alkyl, (C₃-C₁₀)-Cycloalkyl, (C₁-C₆)- Alkylen-(C₆-C₁₀)-Aryl, -(C₆-C₁₀)-Aryl, Heterozyklyl, (C1-C6)-Alkylen-(C3-C₁₀)- Heterozyklyl;
sowie deren physiologisch verträglichen Salze.

5. Verbindungen der Formel I, gemäß einem oder mehreren der Ansprüche 1 bis 4, **dadurch gekennzeichnet, dass** darin bedeuten
R1 H;
R2 (C₁-C₁₀)-Alkyl, (C₃-C₁₀)-Cycloalkyl, Phenyl, (C₁-C₆)-Alkylen-Phenyl, ein Pyrrolidino-, Piperidino-, Hexamethylenimino-, Morpholino-, Piperazino-, Thiomorpholino- oder Homopiperazino-Rest;
sowie deren physiologisch verträglichen Salze.

6. Arzneimittel enthaltend eine oder mehrere der Verbindungen gemäß einem oder mehreren der Ansprüche 1 bis 5.

7. Arzneimittel enthaltend eine oder mehrere der Verbindungen gemäß einem oder mehreren der Ansprüche 1 bis 5 und mindestens einen weiteren Wirkstoff.

8. Arzneimittel, gemäß Anspruch 7, **dadurch gekennzeichnet, daß** es als weiteren Wirkstoff eine oder mehrere Antidiabetika, hypoglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, Fibrate, MTP-Inhibitoren, Gallensäureresorptionsinhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen synthetase inhibitoren, Lipoprotein(a) antagonisten, Lipase Inhibitoren, Insuline, Sulphonylhamstoffe, Biguanide, Meglitinide, Thiazolidindione, α-Glukosidase-Inhibitoren, auf den ATP-abhängigen Kaliumkanal der Betazellen wirkende Wirkstoffe, CART-Agonisten, NPY-Agonisten, MC4-Agonisten, Orexin-Agonisten, Cannabinoid 1 Rezeptor Antagonisten, H3-Agonisten, TNF-Agonisten, CRF-Agonisten, CRF BP-Antagonisten, Urocortin-Agonisten, β3-Agonisten, MSH (Melanocyt-stimulierendes Hormon)-Agonisten, CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, , Cannabinoid 1 Rezeptor Antagonisten , RXR-Modulatoren oder TR-β-Agonisten oder Amphetamine enthält.

9. Verwendung der Verbindungen gemäß einem oder mehreren der Ansprüche 1 bis 5 zur Herstellung eines Medikamentes zur Blutzuckersenkung.

10. Verwendung der Verbindungen gemäß einem oder mehreren der Ansprüche 1 bis 5 zur Herstellung eines Medikamentes zur Behandlung des Typ 2 Diabetes.

11. Verwendung der Verbindungen gemäß einem oder mehreren der Ansprüche 1 bis 5 zur Herstellung eines Medikamentes zur Behandlung von Lipid- und Kohlenhydratstoffwechselstörungen.

12. Verwendung der Verbindungen gemäß einem oder mehreren der Ansprüche 1 bis 5 zur Herstellung eines Medikaments zur Behandlung arteriosklerotischer Erscheinungen.

13. Verwendung der Verbindungen gemäß einem oder mehreren der Ansprüche 1 bis 5 zur Herstellung eines Medikaments zur Behandlung von Insulin Resistenz.

14. Verfahren zur Herstellung eines Arzneimittels enthaltend eine oder mehrere der Verbindungen gemäß einem oder mehreren der Ansprüche 1 bis 5, **dadurch gekennzeichnet, dass** der Wirkstoff mit einem pharmazeutisch geeigneten Träger vermischt wird und diese Mischung in eine für die Verabreichung geeignete Form gebracht wird.

## Claims

1. A compound of the formula I, 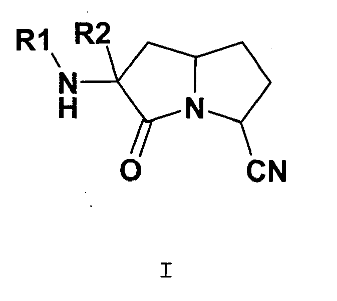 in which the meanings are
R1 H, (C₁-C₁₀)-alkyl, (C₃-C₁₀)-cycloalkyl, (C₂-C₆)- alkenyl, (C₂-C₆)-alkynyl, (C₆-C₁₀)-aryl, heterocyclyl, COR3, COOR3, CONR3R4, CN, where the alkyl, cycloalkyl, alkenyl, alkynyl, aryl and heterocyclyl radicals may be substituted one or more times by F, Cl, Br, I, CN, NO₂, SH, SF₅, OH, (C₁-C₆)-alkyl, -CF₃, (C₂-C₆)- alkenyl, (C₂-C₆)-alkynyl, OR3, OP(O)(OR3)₂, NR3R4, NR3CONR3R4, COR3, OCOR3, OCOOR3, COOR3, CONR3R4, OCONR3R4, (C₁-C₆)-alkylene-OR3, (C₁- C₆)-alkylene-NR3R4, (C₁-C₆)-alkylene-NR3SO₂R4, (C₁-C₆)-alkylene-SR3, alkylene-S(O)R3, alkylene-S(O)₂R3, alkylene-S(O)₂NR3R4, (C₁-C₆)- alkylene-COR3, (C₁-C₆)-alkylene-COOR3 , (C₁-C₆)- alkylene-CONR3R4, SR3, SOR3, SO₂R3, SO₂NR3R4, NR3SO₂R4, (C₁-C₆)-alkylene-(C₃-C₁₀)-cycloalkyl, (C₁-C₆)-alkylene-(C₆-C₁₀)-aryl, (C₁-C₆)- alkylene-heterocyclyl, (C₃-C₁₀)-cycloalkyl, (C₆-C₁₀)-aryl or heterocyclyl;
R2 H, (C₁-C₁₀)-alkyl, (C₃-C₁₀)-cycloalkyl, (C₂-C₆)- alkenyl, (C₂-C₆)-alkynyl, (C₆-C₁₀)-aryl, heterocyclyl, COR3, COOR3, CONR3R4, CN, where the alkyl, cycloalkyl, alkenyl, alkynyl, aryl and heterocyclyl radicals may be substituted one or more times by F, Cl, Br, I, CN, NO₂, SH, SF₅, OH, (C₁-C₆)-alkyl, -CF₃, (C₂-C₆)- alkenyl, (C₂-C₆)-alkynyl, OR3, OP(O)(OR3)₂, NR3R4, NR3CONR3R4, COR3, OCOR3, OCOOR3, COOR3, CONR3R4, OCONR3R4, (C₁-C₆)-alkylene-OR3, (C₁- C₆)-alkylene-NR3R4, (C₁-C₆)-alkylene-NR3SO₂R4, (C₁-C₆)-alkylene-SR3, alkylene-S(O)R3, alkylene-S(O)₂R3, alkylene-S(O)₂NR3R4, (C₁-C₆)- alkylene-COR3, (C₁-C₆)-alkylene-COOR3, (C₁-C₆)- alkylene-CONR3R4, SR3, SOR3, SO₂R3, SO₂NR3R4, NR3SO₂R4, (C₁-C₆)-alkylene- (C₃-C₁₀)-cycloalkyl, (C₁-C₆)-alkylene-(C₆-C₁₀)-aryl, (C₁-C₆)- alkylene-heterocyclyl, (C₃-C₁₀)-cycloalkyl, (C₆-C₁₀)-aryl or heterocyclyl;
R3, R4 independently of one another H, (C₁-C₆)-alkyl, -CF₃, (C₃-C₁₀)-cycloalkyl, (C₆-C₁₀)-aryl, heterocyclyl, (C₁-C₆)-alkylene-CONR5R6, CONR5R6, (C₁-C₆)-alkylene-COOR5, COOR5, COR5, (C₁-C₆)-alkylene-COR5, (C₁-C₆)-alkylene-OR5, (C₁-C₆)-alkylene-NR5R6, (C₁-C₆)-alkylene-SR5, (C₁-C₆)-alkylene-S(O)R5, (C₁-C₆)-alkylene- S(O)₂R5, S(O)R5, S(O)₂R5, (C₁-C₄)-alkylene-(C₆- C₁₀)-aryl, or (C₁-C₄)-alkylene-heterocyclyl,
R5, R6 independently of one another H, (C₁-C₆)-alkyl, (C₁-C₆)-alkylene-(C₆-C₁₀)-aryl, - (C₆-C₁₀)-aryl, heterocyclyl, (C₁-C₆)-alkylene-heterocyclyl;
and the physiologically tolerated salts thereof.

2. The compound of the formula I, as claimed in claim 1, **characterized in that** the meanings therein are
R1 H
R2 H, (C₁-C₁₀)-alkyl, (C₃-C₁₀)-cycloalkyl, (C₂-C₆)- alkenyl, (C₂-C₆)-alkynyl, (C₆-C₁₀)-aryl , heterocyclyl, COR3, COOR3, CONR3R4, CN, where the alkyl, cycloalkyl, alkenyl, alkynyl, aryl and heterocyclyl radicals may be substituted one or more times by F, Cl, Br, I, CN, NO₂, SH, SF₅, OH, (C₁-C₆)-alkyl, -CF₃, (C₂-C₆)- alkenyl, (C₂-C₆)-alkynyl, OR3, OP(O)(OR3)₂, NR3R4, NR3CONR3R4, COR3, OCOR3, OCOOR3, COOR3, CONR3R4, OCONR3R4, (C₁-C₆)-alkylene-OR3, (C₁- C₆)-alkylene-NR3R4, (C₁-C₆)-alkylene-NR3SO₂R4, (C₁-C₆)-alkylene-SR3, alkylene-S(O)R3, alkylene-S(O)₂R3, alkylene-S(O)₂NR3R4, (C₁-C₆)- alkylene-COR3, (C₁-C₆)-alkylene-COOR3, (C₁-C₆)- alkylene-CONR3R4, SR3, SOR3, SO₂R3, SO₂NR3R4, NR3SO₂R4, (C₁-C₆)-alkylene-(C₃-C₁₀)-cycloalkyl, (C₁-C₆)-alkylene-(C₆-C₁₀)-aryl, (C₁-C₆)- alkylene-heterocyclyl, (C₃-C₁₀)-cycloalkyl, (C₆-C₁₀)-aryl or heterocyclyl;
R3, R4 independently of one another H, (C₁-C₆)-alkyl, -CF₃, (C₃-C₁₀)-cycloalkyl, (C₆-C₁₀)-aryl, heterocyclyl, (C₁-C₆)-alkylene-CONR5R6, CONR5R6, (C₁-C₆)-alkylene-COOR5, COOR5, COR5, (C₁-C₆)-alkylene-COR5, (C₁-C₆)-alkylene-OR5, (C₁-C₆)-alkylene-NR5R6, (C₁-C₆)-alkylene-SR5, (C₁-C₆)-alkylene-S(O)R5, (C₁-C₆)-alkylene- S(O)₂R5, S(O)R5, S(O)₂R5, (C₁-C₄)-alkylene-(C₆- C₁₀)-aryl, or (C₁-C₄)-alkylene-heterocyclyl,
R5, R6 independently of one another H, (C₁-C₆)-alkyl, (C₁-C₆)-alkylene-(C₆-C₁₀)-aryl, -(C₆-C₁₀)-aryl, heterocyclyl, (C₁-C₆)-alkylene-heterocyclyl;
and the physiologically tolerated salts thereof.

3. The compound of the formula I, as claimed in claim 1 or 2, **characterized in that** the meanings therein are
R1 H
R2 (C₁-C₁₀)-alkyl, (C₃-C₁₀)-cycloalkyl, (C₂-C₆)- alkenyl, (C₂-C₆)-alkynyl, (C₆-C₁₀)-aryl, heterocyclyl, COR3, COOR3, CONR3R4, CN, where the alkyl, cycloalkyl, alkenyl, alkynyl, aryl and heterocyclyl radicals may be substituted one or more times by F, Cl, Br, I, CN, NO₂, SH, SF₅, OH, (C₁-C₆)-alkyl, -CF₃, (C₂-C₆)- alkenyl, (C₂-C₆)-alkynyl, OR3, OP(O)(OR3)₂, NR3R4, NR3CONR3R4, COR3, OCOR3, OCOOR3, COOR3, CONR3R4, OCONR3R4, (C₁-C₆)-alkylene-OR3, (C₁- C₆)-alkylene-NR3R4, (C₁-C₆)-alkylene-NR3SO₂R4, (C₁-C₆)-alkylene-SR3, alkylene-S(O)R3, alkylene-S(O)₂R3, alkylene-S(O)₂NR3R4, (C₁-C₆)- alkylene-COR3, (C₁-C₆)-alkylene-COOR3, (C₁-C₆)- alkylene-CONR3R4, SR3, SOR3, SO₂R3, SO₂NR3R4, NR3SO₂R4, (C₁-C₆)-alkylene-(C₃-C₁₀)-cycloalkyl, (C₁-C₆)-alkylene-(C₆-C₁₀)-aryl, (C₁-C₆)- alkylene-heterocyclyl, (C₃-C₁₀)-cycloalkyl, (C₆-C₁₀)-aryl or heterocyclyl;
R3, R4 independently of one another H, (C₁-C₆)-alkyl, -CF₃, (C₃-C₁₀)-cycloalkyl, (C₆-C₁₀)-aryl, heterocyclyl, (C₁-C₆)-alkylene-CONR5R6, CONR5R6, (C₁-C₆)-alkylene-COOR5, COOR5, COR5, (C₁-C₆)-alkylene-COR5, (C₁-C₆)-alkylene-OR5, (C₁-C₆)-alkylene-NR5R6, (C₁-C₆)-alkylene-SR5, (C₁-C₆)-alkylene-S(O)R5, (C₁-C₆)-alkylene- S(O)₂R5, S(O)R5, S(O)₂R5, (C₁-C₄)-alkylene-(C₆- C₁₀)-aryl, or (C₁-C₄)-alkylene-heterocyclyl,
R5, R6 independently of one another H, (C₁-C₆)-alkyl, (C₁-C₆)-alkylene-(C₆-C₁₀)-aryl, -(C₆-C₁₀)-aryl, heterocyclyl, (C₁-C₆)-alkylene-heterocyclyl;
and the physiologically tolerated salts thereof.

4. A compound of the formula I as claimed in one or more of claims 1 to 3, **characterized in that** the meanings therein are
R1 H;
R2 (C₁-C₁₀)-alkyl, (C₃-C₁₀)-cycloalkyl, (C₂-C₆)- alkenyl, (C₂-C₆)-alkynyl, (C₆-C₁₀)-aryl, a pyrrolidino, piperidino, hexamethyleneimino, morpholino, piperazino, thiomorpholino or homopiperazino radical; where the alkyl, cycloalkyl, alkenyl, alkinyl, pyrrolidino, piperidino, hexamethyleneimino, morpholino, piperazino, thiomorpholino, homopiperazino and aryl radicals may be substituted one or more times by F, Cl, Br, CN, SF₅, OH, (C₁-C₆)-alkyl, -CF₃, (C₂-C₆)-alkenyl, OR3, NR3R4, NR3CONR3R4, COR3, OCOR3, CO₂R3, CONR3R4, OCONR3R4, (C₁-C₆)- alkylene-OR3, (C₁-C₆)-alkylene-NR3R4, (C₁-C₆)- alkylene-NR3SO₂R4, (C₁-C₆)-alkylene-SR3, alkylene-S(O)R3, alkylene-S(O)₂R3, alkylene- S(O)₂NR3R4, (C₁-C₆)-alkylene-COR3, (C₁-C₆)- alkylene-CO₂R3, (C₁-C₆)-alkylene-CONR3R4, SR3, SOR3, SO₂R3, SO₂NR3R4, NR3SO₂R4, (C₁-C₆)- alkylene-(C₃-C₁₀)-cycloalkyl, (C₁-C₆)-alkylene- (C₆-C₁₀)-aryl, (C₁-C₆)-alkylene-heterocyclyl, (C₃-C₁₀)-cycloalkyl, (C₆-C₁₀)-aryl which may be substituted one or more times by F, Cl, Br, I, CN, OH, -CF₃, (C₁-C₆)-alkyl, OR3, NR3R4, COR3, CO2R3, CONR3R4, or heterocyclyl which may be substituted one or more times by F, Cl, Br, CN, NO₂, OH, -CF₃, (C₁-C₆)-alkyl, OR3, NR3R4, COR3, CO2R3, CONR3R4;
R3, R4 independently of one another H, (C₁-C₆)-alkyl, -CF₃, (C₃-C₁₀)-cycloalkyl, (C₆-C₁₀)-aryl, heterocyclyl, (C₁-C₆)-alkylene-CONR5R6, (C₁- C₆)-alkylene-COOR5, (C₁-C₆)-alkylene-COR5, (C₁-C₆)-alkylene-OR5, (C₁-C₆)-alkylene-NR5R6, (C1-C6)-alkylene-SR5, (C₁-C₆)-alkylene-S(O)R5, (C₁-C₆)-alkylene-S(O)₂R5, (C₁-C₄)-alkylene-(C₆- C₁₀)-aryl or (C₁-C₄)-alkylene-heterocyclyl;
R5, R6 independently of one another H, (C₁-C₆)-alkyl, (C₃-C₁₀)-cycloalkyl, (C₁-C₆)-alkylene-(C₆-C₁₀)- aryl, -(C₆-C₁₀)-aryl, heterocyclyl, (C1-C6)- alkylene-(C₃-C₁₀)-heterocyclyl;
and the physiologically tolerated salts thereof.

5. A compound of the formula I as claimed in one or more of claims 1 to 4, **characterized in that** the meanings therein are
R1 H;
R2 (C₁-C₁₀)-alkyl, (C₃-C₁₀)-cycloalkyl, phenyl, (C₁-C₆)-alkylene-phenyl, a pyrrolidino, piperidino, hexamethyleneimino, morpholino, piperazino, thiomorpholino or homopiperazino radical;
and the physiologically tolerated salts thereof.

6. A medicament comprising one or more of the compounds as claimed in one or more of claims 1 to 5.

7. A medicament comprising one or more of the compounds as claimed in one or more of claims 1 to 5 and at least one other active ingredient.

8. The medicament as claimed in claim 7, **characterized in that** it comprises as other active ingredient one or more antidiabetics, hypoglycemic active ingredients, HMGCoA reductase inhibitors, cholesterol absorption inhibitors, PPAR gamma agonists, PPAR alpha agonists, PPAR alpha/gamma agonists, fibrates, MTP inhibitors, bile acid absorption inhibitors, CETP inhibitors, polymeric bile acid adsorbents, LDL receptor inducers, ACAT inhibitors, antioxidants, lipoprotein lipase inhibitors, ATP-citrate lyase inhibitors, squalene synthetase inhibitors, lipoprotein(a) antagonists, lipase inhibitors, insulins, sulfonylureas, biguanides, meglitinides, thiazolidinediones, α-glucosidase inhibitors, active ingredients which act on the ATP-dependent potassium channel of the beta cells, CART agonists, NPY agonists, MC4 agonists, orexin agonists, cannabinoid 1 receptor antagonists, H3 agonists, TNF agonists, CRF agonists, CRF BP antagonists, urocortin agonists, β3 agonists, MSH (melanocyte-stimulating hormone) agonists, CCK agonists, serotonin reuptake inhibitors, mixed sertoninergic and noradrenergic compounds, 5HT agonists, bombesin agonists, galanin antagonists, growth hormones, growth hormone-releasing compounds, TRH agonists, uncoupling protein 2 or 3 modulators, leptin agonists, DA agonists (bromocriptine, Doprexin), lipase/amylase inhibitors, PPAR modulators, cannabinoid 1 receptor antagonists, RXR modulators or TR-β agonists or amphetamines.

9. The use of the compounds as claimed in one or more of claims 1 to 5 for producing a medicament for reducing blood glucose.

10. The use of the compounds as claimed in one or more of claims 1 to 5 for producing a medicament for the treatment of type 2 diabetes.

11. The use of the compounds as claimed in one or more of claims 1 to 5 for producing a medicament for the treatment of disturbances of lipid and carbohydrate metabolism.

12. The use of the compounds as claimed in one or more of claims 1 to 5 for producing a medicament for the treatment of arteriosclerotic manifestations.

13. The use of the compounds as claimed in one or more of claims 1 to 5 for producing a medicament for the treatment of insulin resistance.

14. A process for producing a medicament comprising one or more of the compounds as claimed in one or more of claims 1 to 5, **characterized in that** the active ingredient is mixed with a pharmaceutically suitable carrier and this mixture is converted into a form suitable for administration.

## Revendications

1. Composés de formule I, 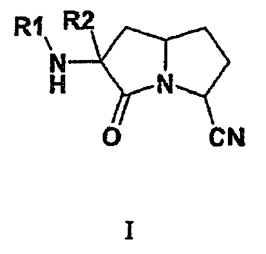 où
R1 signifie H, (C₁-C₁₀)-alkyle, (C₃-C₁₀)-cycloalkyle, (C₂-C₆)-alcényle, (C₂-C₆)-alcynyle, (C₆-C₁₀)-aryle, hétérocyclyle, COR3, COOR3, CONR3R4, CN, où les radicaux alkyle, cycloalkyle, alcényle, alcynyle, aryle et hétérocyclyle peuvent être monosubstitués ou polysubstitués par F, Cl, Br, I, CN, NO₂, SH, SF₅, OH, (C₁-C₆)-alkyle, CF₃, (C₂-C₆)-alcényle, (C₂- C₆) alcynyle,OR3, OP(O)(OR3)₂ , NR3R4, NR3CONR3R4, COR3, OCOR3,OCOOR3, COOR3, CONR3R4, OCONR3R4, (C₁- C₆)-alkylène-OR3, (C₁-C₆) -alkylène-NR3R4, (C₁-C₆)- alkylène-NR3SO₂R4, (C₁-C₆)-alkylène-SR3, alkylène-S (O)R3,alkylène-S(O)₂R3, alkylène-S(O)₂NR3R4, (C₁- C₆)-alkylène-COR3, (C₁-C₆)-alkylène-COOR3, (C₁-C₆)- alkylène-CONR3R4, SR3, SOR3, SO₂R3, SO₂NR3R4, NR3SO₂R₄, (C₁-C₆)-alkylène- (C₃-C₁₀)-cycloalkyle, (C₁- C₆)-alkylène-(C₆-C₁₀)-aryle, (C₁-C₆)-alkylène- hétérocyclyle, (C₃-C₁₀)-cycloalkyle, (C₆-C₁₀) -aryle ou hétérocyclyle ;
R2 signifie H, (C₁-C₁₀)-alkyle, (C₃-C₁₀)-cycloalkyle, (C₂-C₆)-alcényle, (C₂-C₆)-alcynyle, (C₆-C₁₀)-aryle, hétérocyclyle, COR3, COOR3, CONR3R4, CN, où les radicaux alkyle, cycloalkyle, alcényle, alcynyle, aryle et hétérocyclyle peuvent être monosubstitués ou polysubstitués par F, Cl, Br, I, CN, NO₂, SH, SF₅,OH, (C₁-C₆)-alkyle, CF₃, (C₂-C₆)-alcényle, (C₂- C₆)-alcynyle, OR₃, OP(O)(OR3)₂, NR3R4, NR3CONR3R4, COR3, OCOR3, OCOOR3, COOR3, CONR3R4, OCONR3R4, (C₁- C₆)-alkylène-OR3, (C₁-C₆)-alkylène-NR3R4, (C₁-C₆)- alkylène-NR3SO₂R4, (C₁-C₆)-alkylène-SR3, alkylène-S (O)R3, alkylène-S(O)₂R3, alkylène-S(O)₂NR3R4, (C₁- C₆)-alkylène-COR3, (C₁-C₆)-alkylène-COOR3, (C₁-C₆)- alkylène-CONR3R4, SR3, SOR3, SO₂R3, SO₂NR3R4, NR3SO₂R4,(C₁-C₆)-alkylène-(C₃-C₁₀)-cycloalkyle, (C₁- C₆)-alkylène-(C₆-C₁₀)-aryle, (C₁-C₆)-alkylène- hétérocyclyle, (C₃-C₁₀)-cycloalkyle, (C₆-C₁₀)-aryle ou hétérocyclyle ;
R3, R4 signifient, indépendamment l'un de l'autre H, (C₁-C₆)-alkyle, CF₃, (C₃-C₁₀)-cycloalkyle, (C₆-C₁₀)- aryle, hétérocyclyle, (C₁-C₆)-alkylène-CONR5R6, CONR5R6, (C₁-C₆)-alkylène-COOR5, COOR5, COR5, (C₁- C₆) -alkylène-COR5, (C₁-C₆)-alkylène-OR5, (C₁-C₆)- alkylène-NR5R6,(C₁-C₆)-alkylène-SR5, (C₁-C₆)- alkylène-S(O)R5, (C₁-C₆)-alkylène-S(O₂R5, S(O)R5, S(O)₂R5, (C₁-C₄) -alkylène- (C₆-C₁₀) -aryle ou (C₁-C₄)- alkylène-hétérocyclyle ;
R5, R6 signifient, indépendamment l'un de l'autre, H, (C₁-C₆)-alkyle, (C₁-C₆)-alkylène- (C₆-C₁₀)-aryle, (C₆-C₁₀)-aryle, hétérocyclyle, (C₁-C₆)-alkylène- hétérocyclyle ;
ainsi que leurs sels physiologiquement acceptables.

2. Composés de formule I selon la revendication 1, **caractérisés en ce que**
R1 signifie H
R2 signifie H, (C₁-C₁₀)-alkyle, (C₃-C₁₀)-cycloalkyle, (C₂-C₆)-alcényle, (C₂-C₆)-alcynyle, (C₆-C₁₀)-aryle, hétérocyclyle, COR3, COOR3, CONR3R4, CN, où les radicaux alkyle, cycloalkyle, alcényle, alcynyle, aryle et hétérocyclyle peuvent être monosubstitués ou polysubstitués par F, Cl, Br, I, CN, NO₂, SH, SF₅, OH, (C₁-C₆)-alkyle, CF₃, (C₂-C₆)-alcényle, (C₂- C₆)-alcynyle, OR3, OP(O)(OR3)₂, NR3R4, NR3CONR3R4, COR3, OCOR3, OCOOR3, COOR3, CONR3R4, OCONR3R4, (C₁- C₆)-alkylène-OR3, (C₁-C₆)-alkylène-NR3R4, (C₁-C₆)- alkylène-NR3SO₂R4, (C₁-C₆)-alkylène-SR3, alkylène-S (O)R3,alkylène-S(O)₂R3,alkylène-S(O)₂NR3R4, (C₁- C₆)-alkylène-COR3, (C₁-C₆)-alkylène-COOR3, (C₁-C₆)- alkylène-CONR3R4, SR3, SOR3, SO₁R3, SO₂NR3R4, NR3SO₂R4, (C₁-C₆) -alkylène- (C₃-C₁₀) -cycloalkyle, (C₁- C₆)-alkylène-(C₆-C₁₀)-aryle, (C₁-C₆)-alkylène- hétérocyclyle,(C₃-C₁₀) -cycloalkylen, (C₆-C₁₀)-aryle ou hétérocyclyle ;
R3, R4 signifient, indépendamment l'un de l'autre H, (C₁-C₆)-alkyle, CF₃, (C₃-C₁₀) -cycloalkyle, (C₆-C₁₀)- aryle, hétérocyclyle, (C₁-C₆)-alkylène-CONR5R6, CONR5R6, (C₁-C₆)-alkylène-COOR5, COOR5, COR5, (C₁- C₆)-alkylène-COR5, (C₁-C₆)-alkylène-OR5, (C₁-C₆)- alkylène-NR5R6, (C₁-C₆)-alkylène-SR5, (C₁-C₆)- alkylène-S(O)R5, (C₁-C₆)-alkylène-S(O)₂R5, S(O)R5, S(O)₂R5, (C₁-C₄) -alkylène- (C₆-C₁₀) -aryle ou (C₁-C₄)- alkylène-hétérocyclyle ;
R5, R6 signifient, indépendamment l'un de l'autre, H, (C₁-C₆)-alkyle, (C₁-C₆)-alkylène- (C₆-C₁₀)-aryle, (C₆-C₁₀)-aryle, hétérocyclyle, (C₁-C₆)-alkylène- hétérocyclyle ;
ainsi que leurs sels physiologiquement acceptables.

3. Composés de formule I selon la revendication 1 ou 2, **caractérisés en ce que**
R1 signifie H
R2 signifie (C₁-C₁₀)-alkyle, (C₃-C₁₀)-cycloalkyle, (C₂- C₆)-alcényle, (C₂-C₆)-alcynyle, (C₆-C₁₀)-aryle, hétérocyclyle, COR3, COOR3, CONR3R4, CN, où les radicaux alkyle, cycloalkyle, alcényle, alcynyle, aryle et hétérocyclyle peuvent être monosubstitués ou polysubstitués par F, Cl, Br, I, CN, NO₂, SH, SF₅, OH, (C₁-C₆)-alkyle, CF₃, (C₂-C₆)-alcényle, (C₂- C₆)-alcynyle, OR3, OP (O) (OR3)₂, NR3R4, NR3CONR3R4, COR3, OCOR3, OCOOR3, COOR3, CONR3R4, OCONR3R4, (C₁- C₆)-alkylène-OR3,(C₁-C₆)-alkylène-NR3R4, (C₁-C₆)- alkylène-NR3SO₂R4, (C₁-C₆)-alkylène-SR3, alkylène-S (O)R3,alkylène-S(O)₂R3,alkylène-S(O)₂NR3R4, (C₁- C₆)-alkylène-COR3, (C₁-C₆)-alkylène-COOR3, (C₁-C₆)- alkylène-CONR3R4, SR3, SOR3, SO₂R3, SO₂NR3R4, NR3SO₂R4, (C₁-C₆)-alkylène- (C₃-C₁₀) -cycloalkyle, (C₁- C₆)-alkylène-(C₆-C₁₀)-aryle, (C₁-C₆)-alkylène- hétérocyclyle,(C₃-C₁₀)-cycloalkyle, (C₆-C₁₀) -aryle ou hétérocyclyle ;
R3_{f} R4 signifient, indépendamment l'un de l'autre H, (C₁-C₆) -alkyle, CF₃, (C₃-C₁₀)-cycloalkyle, (C₆-C₁₀)- aryle, hétérocyclyle, (C₁-C₆)-alkylène-CONR5R6, CONR5R6, (C₁-C₆)-alkylène-COOR5, COOR5, COR5, (C₁- C₆)-alkylène-COR5, (C₁-C₆)-alkylène-OR5, (C₁-C₆)- alkylène-NR5R6, (C₁-C₆)-alkylène-SR5, (C₁-C₆)- alkylène-S(O)R5, (C₁-C₆)-alkylène-S(O)₂R5, S(O)R5, S(O)₂R5, (C₁-C₄)-alkylène-(C₆-C₁₀)-aryle ou (C₁-C₄)- alkylène-hétérocyclyle ;
R5, R6 signifient, indépendamment l'un de l'autre, H, (C₁-C₆)-alkyle, (C₁-C₆)-alkylène- (C₆-C₁₀)-aryle, (C₆-C₁₀)-aryle, hétérocyclyle, (C₁-C₆)-alkylène- hétérocyclyle ;
ainsi que leurs sels physiologiquement acceptables.

4. Composés de formule I selon l'une ou plusieurs des revendications 1 à 3, **caractérisés en ce que**
R1 signifie H ;
R2 signifie (C₁-C₁₀)-alkyle, (C₃-C₁₀)-cycloalkyle, (C₂- C₆)-alcényle, (C₂-C₆)-alcynyle, (C₆-C₁₀)-aryle, un radical pyrrolidino, pipéridino, hexaméthylène- imino, morpholino, pipérazino, thiomorpholino ou homopipérazino ; où les radicaux alkyle, cycloalkyle, alcényle, alcynyle, pyrrolidino, pipéridino, hexaméthylène-imino, morpholino, pipérazino, thiomorpholino, homopipérazino et aryle peuvent être monosubstitués ou polysubstitués par F, Cl, Br, CN, SF₅, OH, (C₁-C₆)- alkyle, CF₃, (C₂-C₆)-alcényle, OR3, NR3R4, NR3CONR3R4, COR3, OCOR3, CO₂R3, CONR3R4, OCONR3R4, (C₁-C₆)-alkylène-OR3, (C₁-C₆)-alkylène-NR3R4, (C₁- C₆)-alkylène-NR3SO₂R4, (C₁-C₆)-alkylène-SR3, alkylène-S(O)R3, alkylène-S(O)₂R3, alkylène-S(O) ₂NR3R4, (C₁-C₆)-alkylène-COR3, (C₁-C₆)-alkylène- CO₂R3, (C₁-C₆)-alkylène-CONR3R4, SR3, SOR3, SO₂R3, SO₂NR3R4, NR3SO₂R4, (C₁-C₆)-alkylène-(C₃-C₁₀)- cycloalkyle, (C₁-C₆)-alkylène- (C₆-C₁₀)-aryle, (C₁- C₆)-alkylène-hétérocyclyle, (C₃-C₁₀)-cycloalkyle, (C₆-C₁₀)-aryle, qui peut être monosubstitué ou polysubstitué par F, Cl, Br, I, CN, OH, CF₃, (C₁- C₆)-alkyle, OR3, NR3R4, COR3, CO₂R3, CONR3R4 ; ou hétérocyclyle qui peut être monosubstitué ou polysubstitué par F, Cl, Br, CN, NO₂, OH, CF₃, (C₁- C₆)-alkyle, OR3, NR3R4, COR3, CO₂R3, CONR3R4 ;
R3, R4 signifient, indépendamment l'un de l'autre H, (C₁-C₆) -alkyle, CF₃, (C₃-C₁₀)-cycloalkyle, (C₆-C₁₀)- aryle, hétérocyclyle, (C₁-C₆)-alkylène-CONR5R6, (C₁-C₆) -alkylène-COOR5, (C₁-C₆)-alkylène-COR5, (C₁- C₆)-alkylène-OR5,(C₁-C₆)-alkylène-NR5R6, (C₁-C₆)- alkylène-SR5, (C₁-C₆)-alkylène-S(O)R5, (C₁-C₆)- alkylène-S(O)₂R5, (C₁-C₄)-alkylène-(C₆-C₁₀)-aryle ou (C₁-C₄)-alkylène-hétérocyclyle ;
R5, R6 signifient, indépendamment l'un de l'autre, H, (C₁-C₆)-alkyle, (C₃-C₁₀)-cycloalkyle, (C₁-C₆)- alkylène-(C₆-C₁₀)-aryle, -(C₆-C₁₀)-aryle, hétérocyclyle, (C₁-C₆)-alkylène-(C₃-C₁₀)- hétérocyclyle ;
ainsi que leurs sels physiologiquement acceptables.

5. Composés de formule I selon l'une ou plusieurs des revendications 1 à 4, **caractérisés en ce que**
R1 signifie H ;
R2 signifie (C₁-C₁₀)-alkyle, (C₃-C₁₀)-cycloalkyle, phényle,(C₁-C₆)-alkylène-phényle, un radical pyrrolidino, pipéridino, hexaméthylène-imino, morpholino, pipérazino, thiomorpholino ou homopipérazino ;
ainsi que leurs sels physiologiquement acceptables.

6. Médicament contenant un ou plusieurs des composés selon l'une ou plusieurs des revendications 1 à 5.

7. Médicament contenant un ou plusieurs composés selon l'une ou plusieurs des revendications 1 à 5 et au moins une autre substance active.

8. Médicament selon la revendication 7, **caractérisé en ce qu'**il contient comme autre substance active un(e) ou plusieurs antidiabétiques, substances actives hypoglycémiques, inhibiteurs de la HMGCoA-réductase, inhibiteurs de la résorption de cholestérol, agonistes de PPAR gamma, agonistes de PPAR alpha, agonistes de PPAR alpha/gamma, fibrates, inhibiteurs de MTP, inhibiteurs de la résorption de l'acide biliaire, inhibiteurs de CETP, adsorbants polymères de l'acide biliaire, inducteurs de récepteurs de LDL, inhibiteurs d'ACAT, antioxydants, inhibiteurs de la lipoprotéine-lipase, inhibiteurs de l'ATP-citrate lyase, inhibiteurs de la squalène-synthétase, antagonistes de la lipoprotéine (a), inhibiteurs de lipase, insulines, sulfonylurées, biguanides, méglitinides, thiazolidinediones, inhibiteurs de l'α-glucosidase, substances actives agissant sur le canal potassique dépendant d'ATP des cellules bêta, agonistes de CART, antagonistes de NPY, agonistes de MC₄, agonistes d'orexine, antagonistes du récepteur des cannabinoïdes 1, agonistes de H3, agonistes de TNF, agonistes de CRF, antagonistes de CRF BP, agonistes d'urocortine, agonistes de ß3, agonistes de MSH (hormone stimulant les mélanocytes), agonistes de CCK, inhibiteurs de la résorption de la sérotonine, composés sérotoninergiques et noradrénergiques mixtes, agonistes de 5HT, agonistes de bombésine, agonistes de galanine, hormones de croissance, composés libérant l'hormone de croissance, agonistes de TRH, modulateurs 2 ou 3 de la protéine découplante, agonistes de leptine, agonistes de DA (Bromocriptine, Doprexine), inhibiteurs de lipase/amylase, modulateurs de PPAR, antagonistes du récepteur des cannabinoïdes 1, modulateurs de RXR ou agonistes de TR-ß ou amphétamines.

9. Utilisation des composés selon l'une ou plusieurs des revendications 1 à 5 pour la préparation d'un médicament destiné à abaisser la glycémie.

10. Utilisation des composés selon l'une ou plusieurs des revendications 1 à 5 pour la préparation d'un médicament destiné au traitement du diabète de type 2.

11. Utilisation des composés selon l'une ou plusieurs des revendications 1 à 5 pour la préparation d'un médicament destiné au traitement des troubles du métabolisme lipidique et des hydrates de carbone.

12. Utilisation des composés selon l'une ou plusieurs des revendications 1 à 5 pour la préparation d'un médicament destiné au traitement de phénomènes artérioscléreux.

13. Utilisation des composés selon l'une ou plusieurs des revendications 1 à 5 pour la préparation d'un médicament destiné au traitement de la résistance à l'insuline.

14. Procédé pour la préparation d'un médicament contenant un ou plusieurs des composés selon l'une ou plusieurs des revendications 1 à 5, **caractérisé en ce que** la substance active est mélangée avec un support pharmaceutiquement approprié et ce mélange est amené dans une forme appropriée pour l'administration.
